# EUROPEAN PATENT APPLICATION

(11) **EP 1 862 180 A1**
(43) Date of publication of application: **05.12.2007**
(21) Application number: 06730732.2
(22) Date of filing: 24.03.2006
(51) Int. Cl.: A61K 45/00

(54) **PROPHYLACTIC/THERAPEUTIC AGENT FOR CANCER**

(30) Priority: 25.03.2005 JP 2005089613
(71) Applicant: Takeda Pharmaceutical Company Limited, Osaka-shi, Osaka 541-0045 (JP)
(72) Inventor: TOJO, Hideaki, Tsukuba-shi, Ibaraki 300-4293 (JP); SHIBATA, Sachio, Tsukuba-shi, Ibaraki 300-4293 (JP); HORIKOSHI, Kenichi, Tsukuba-shi, Ibaraki, 300-4293 (JP)
(74) Representative: von Kreisler Selting Werner
(86) International application number: PCT/JP2006/306784
(87) International publication number: WO 2006/101273

(57) **Abstract**

The present invention provides a prophylactic/therapeutic agent for cancer or the like, an agent for promoting apoptosis of cancer cells, an agent for suppressing proliferation of cancer cells, and the like, which comprises a compound or its salt that inhibits an activity of a protein comprising the same or substantially the same amino acid sequence as the amino acid sequence represented by SEQ ID NO: 1, a compound or its salt that inhibits the expression of a gene for the protein, an antisense polynucleotide comprising the entirety or part of a nucleotide sequence complementary or substantially complementary to a nucleotide sequence of a polynucleotide encoding the protein or its partial peptide, an antibody against the protein, or the like.

## Description

### TECHNICAL FIELD

The present invention relates to prophylactic/therapeutic agents and diagnostics for cancer, screening of the prophylactic/therapeutic agents for cancer, etc.

### BACKGROUND ART

Studies on morphological formation in the developmental period has been actively performed by screening of variants using drosophia. Hedgehog gene (hh) was found as a gene exhibiting morphological abnormality in the drosophia embryo as a result of variation thereof, and was so named because the morphology of variation was similar to that of hedgehog, which is a small animal. A hedgehog gene product (Hh) is a secreted protein, and is produced as a precursor of about 45 kDa and then is self-decomposed into an N-terminal domain of 20 kDa, which is the main activity part, and a C-terminal domain of 25 kDa. The 20 kDa N-terminal domain as the main activity part is modified with fatty acid at the N-terminus and with cholesterol at the C-terminus. A hedgehog signal transduction system is formed of the following group of proteins. The receptor of Hh is Patched (Ptch), which is a 12-transmembrane type protein. Ptch acts on Smoothened (Smo), which is a seven-transmembrane type protein, and suppresses the function of Smo in the absence of Hh. When Hh is bonded to the receptor Ptch, the suppression on Smo is released to activate Smo. A signal generated by the activation of Smo activates a transcription factor Ci, and thus expression of a group of genes involved in the morphology formation is adjusted (Nybakken & Perrimon, Curr. Opin. Genet. Dev., 12, 503-511, 2002).

In mammals, a gene group corresponding to the hedgehog signal transduction gene group in drosophila has been found, and signal transduction is performed by substantially the same mechanism as that of drosophila. For example, regarding human, three types of genes: sonic hedgehog (Shh), Indian hedgehog (Ihh) and desert hedgehog (Dhh) are known as genes corresponding to hh of drosophila. These genes are modified after translation in substantially the same manner as drosophila Hh. One type of human gene corresponding to Smo and two types of human genes corresponding to Ptch (Ptch1, Ptch2) are known. A human transcription factor corresponding to Ci is considered to be Gli, and three types of Gli, i.e., Gli1, Gli2 nad Gli3, are known (Ruiz et al., Nature Rev. Cancer, 2, 361-372, 2002). In mammals, the hedgehog signal is considered to have an important role in morphological formation in the developmental period as in drosophila. For example, in the case of human, Shh variation has been found in the patient with holoprosencephaly, which is an inborn developmental anomaly (Roessler et al., Nat. Genet., 14, 357-360, 1996). A naturally occurring compound, Cyclopamine (Binns et al., Am. J. Vet. Res., 24, 1164-1175, 1963), extracted from white hellebore as a compound causing cyclopus in ovine was initially not clear in terms of the acting mechanism, but was later confirmed to be a compound inhibiting Smo (Incardona et al, Development, 125, 3553-3562, 1998). Moreover, Shh knockout mouse has been produced. As a phenotype thereof, cyclopus, limb malformation (Chiang et al., Nature, 383, 407-413, 1996) and neural plate malformation (Ma et al., Cell, 111, 63-75, 2002) are known.

Skinny, which is a drosophila gene, is known to suppress the hedgehog signal as a result of variation thereof. Skinny is also called "rasp", and is considered to encode a protein having an acyltransferase-like structure and catalyze the addition of fatty acid to the N-terminus in the mature stage of Hh protein (Chamoun et al., Science, 293, 2080-2084, 2001; Micchelli et al., Development, 129, 843-851, 2002). The N-terminus of human Shh is provided with fatty acid (Pepinsky et al., J. Biol. Chem., 273, 14037-14045, 1998). Since the activity is enhanced by the modification of the N-terminus with fatty acid, such a modification is considered to be necessary to maintain the activity. It has been reported that when palmitic acid was added to E. coli recombinant human Shh with no fatty acid modification, the activity was enhanced 40 times larger, and when myristic acid was added thereto, the activity was enhanced 160 times larger (Taylor et al., Biochemistry, 40, 4359-4371, 2001).

Human gene corresponding to skinny of drosophila is considered to be HHAT (hedgehog acyltransferase) due to the homology thereof. HHAT was first cDNA-cloned as MART-2 (melanoma antigen recognized by T cells 2), which is one of melanoma antigens, but the function thereof in melanoma was not known (Kawakami et al., J. Immun., 166, 2871-2877, 2001). HHAT has MBOAT (membrane bound O-acyltransferase) domain (Hofmann, Trends Biochem. Sci., 25, 111-112, 2000) which is common to acyltransferase such as ACAT (cholesterol acyltransferase), DGAT (diacylglycerol 0-acyltransferase) and the like. Based on this, HHAT is estimated to have an acyltransferase activity on some substrate. In an HHAT knockout mouse, limb malformation was recognized in the prenatal period which is estimated to be derived from the suppression of Shh lipid addition and the suppression of hedgehog signal transduction (Chen et al., Genes Dev., 18, 641-659, 2004).

The relationship between the hedgehog signal and carcinogenesis has been reported regarding some types of cancer (Magliano et al., Nature Rev. Cancer, 3, 903-911, 2003). Shh was increasingly expressed in human pancreatic cancer as the cancer progressed. In a transgenic mouse in which Shh was forcibly expressed in pancreas, variation of K-ras and overexpression of Her2/neu were recognized in pancreas, and PanIN-analogous lesion, which is an initial stage of cancer, appeared (Thayer et al., Nature, 425, 851-856, 2003). In the colorectal cancer tissue, it has been reported that Shh expression is enhanced as compared to in the normal tissue. In basalioma and medulloblastoma, variation of Ptch, Smo and Shh has been identified, which suggests the relationship between the hedgehog signal and these cancers (Cohen, Am. J. Med. Gen., 123A, 5-28, 2003).

Cyclopamine, which is a naturally occurring Smo inhibiting agent, has been reported to have an anti-tumor effect on glioma (Darmane et al., Development, 128, 5201-5212, 2001), medulloblastoma (Berman et al., Science, 297, 1559-1561, 2002), small cell lung cancer (Watkins et al., Nature, 422, 313-317, 2003), pancreatic cancer (Thayer et al., Nature, 425, 851-856, 2003; Berman et al., Nature, 425, 846-851, 2003), bile duct cancer (Berman et al., Nature, 425, 846-851, 2003), prostate cancer (Karhadkar et al., Nature, 431, 707-712, 2004; Sanchez et al., Proc. Natl. Acad. Sci. U.S.A., 101, 12561-12566, 2004), colorectal cancer (Qualtrough et al., Int J. Cancer, 110, 831-837, 2004), and breast cancer (Kubo et al., Cancer Res., 64, 6071-6074, 2004). As a synthetic compound inhibiting Smo, CUR61414 (Williams et al., Proc. Natl. Acad. Sci. U.S.A., 100, 4616-4621, 2003) and SANT-1, 2, 3, 4 (Chen et al., Proc. Natl. Acad. Sci. U.S.A., 99, 14071-14076, 2002) have been reported. It has also been reported that when an anti-Shh antibody was administered to cancer-carrying nude mouse having colorectal cancer cell strain HT-29 planted thereto, the retraction of cancer was recognized, and also a method for inhibiting the proliferation of cells (tumors) using an anti-Shh antibody or a hedgehog antagonist has also been reported (WO 2004/020599).

### DISCLOSURE OF THE INVENTION

A safe preventive/therapeutic agent for cancer having an excellent therapeutic effect is desired.

The Smo activation mechanism at the level of protein structure and the Smo inhibiting mechanism in the low molecular weight compounds are conventionally still unclarified in many points. Therefore, it is expected to be difficult to optimize compounds having a Smo inhibiting action and providing a useful cancer preventing and treating effect.

As a result of active studies for solving the above-described problems, the present inventors found that a compound having an action of inhibiting the HHAT activity, regarding which the enzyme-substrate relationship has been clarified, can be an excellent prophylactic/therapeutic agent for cancer, and completed the present invention.

The present invention provides:
[1] 1. A prophylactic/therapeutic agent for cancer, comprising a compound or its salt that inhibits an activity of a protein comprising the same or substantially the same amino acid sequence as the amino acid sequence represented by SEQ ID NO: 1, its partial peptide, or a salt thereof;
[2] A prophylactic/therapeutic agent for cancer, comprising a compound or its salt that inhibits the expression of a gene for a protein comprising the same or substantially the same amino acid sequence as the amino acid sequence represented by SEQ ID NO: 1, its partial peptide, or a salt thereof;
[3] An antisense polynucleotide, comprising the entirety or part of a nucleotide sequence complementary or substantially complementary to a nucleotide sequence of a polynucleotide encoding a protein comprising the same or substantially the same amino acid sequence as the amino acid sequence represented by SEQ ID NO: 1, or its partial peptide;
[4] A pharmaceutical agent, comprising the antisense polynucleotide according to [3];
[5] The pharmaceutical agent according to [4], which is a prophylactic/therapeutic agent for cancer;
[6] A pharmaceutical agent, comprising an siRNA or an shRNA against a polynucleotide encoding a protein comprising the same or substantially the same amino acid sequence as the amino acid sequence represented by SEQ ID NO: 1, or its partial peptide;
[7] The pharmaceutical agent according to [6], which is a prophylactic/therapeutic agent for cancer;
[8] An antibody against a protein comprising the same or substantially the same amino acid sequence as the amino acid sequence represented by SEQ ID NO: 1, its partial peptide, or a salt thereof;
[9] A prophylactic/therapeutic agent for cancer, comprising the antibody according to [8];
[10] A diagnostic agent for cancer, comprising the antibody according to [8];
[11] A diagnostic agent for cancer, comprising a polynucleotide encoding a protein comprising the same or substantially the same amino acid sequence as the amino acid sequence represented by SEQ ID NO: 1, or its partial peptide;
[12] A method for diagnosing cancer, which comprises using the antibody according to [8] or a polynucleotide encoding a protein comprising the same or substantially the same amino acid sequence as the amino acid sequence represented by SEQ ID NO: 1, or its partial peptide;
[13] Use of a protein comprising the same or substantially the same amino acid sequence as the amino acid sequence represented by SEQ ID NO: 1, its partial peptide, or a salt thereof, as a diagnostic marker for cancer;
[14] A method for screening a prophylactic/therapeutic pharmaceutical agent for cancer, which comprises using a protein comprising the same or substantially the same amino acid sequence as the amino acid sequence represented by SEQ ID NO: 1, its partial peptide, or a salt thereof;
[15] A kit for screening a prophylactic/therapeutic pharmaceutical agent for cancer, comprising a protein comprising the same or substantially the same amino acid sequence as the amino acid sequence represented by SEQ ID NO: 1, its partial peptide, or a salt thereof;
[16] A method for screening a prophylactic/therapeutic pharmaceutical agent for cancer, which comprises using a polynucleotide encoding a protein comprising the same or substantially the same amino acid sequence as the amino acid sequence represented by SEQ ID NO: 1, or its partial peptide;
[17] A kit for screening a prophylactic/therapeutic pharmaceutical agent for cancer, comprising a polynucleotide encoding a protein comprising the same or substantially the same amino acid sequence as the amino acid sequence represented by SEQ ID NO: 1, or its partial peptide;
[18] A method for screening a prophylactic/therapeutic pharmaceutical agent for cancer, which comprises measuring an activity of a protein comprising the same or substantially the same amino acid sequence as the amino acid sequence represented by SEQ ID NO: 1, its partial peptide, or a salt thereof;
[19] A method for screening a prophylactic/therapeutic pharmaceutical agent for cancer, which comprises measuring an amount of a protein comprising the same or substantially the same amino acid sequence as the amino acid sequence represented by SEQ ID NO: 1, its partial peptide, or a salt thereof;
[19a] A method for screening a compound or its salt which inhibits an activity of a protein comprising the same or substantially the same amino acid sequence as the amino acid sequence represented by SEQ ID NO: 1, its partial peptide, or a salt thereof, which comprises measuring and comparing the radioactivity or the fluorescent intensity derived from the palmitic acid or the myristic acid added to a peptide having an amino acid sequence comprised of the 24th to the 197th amino acids of the amino acid sequence represented by SEQ ID NO: 22, or a partial peptide thereof, in the case where (i) (a) the protein comprising the same or substantially the same amino acid sequence as the amino acid sequence represented by SEQ ID NO: 1, its partial peptide, or a salt thereof, (b) the peptide having an amino acid sequence comprised of the 24th to the 197th amino acids of the amino acid sequence represented by SEQ ID NO: 22, or a partial peptide thereof; etc., and (c) radiolabeled or fluorescence-labeled palmitic acid or myristic acid are reacted, and in the case where (ii) (a) the protein comprising the same or substantially the same amino acid sequence as the amino acid sequence represented by SEQ ID NO: 1, its partial peptide, or a salt thereof, (b) the peptide having an amino acid sequence comprised of the 24th to the 197th amino acids of the amino acid sequence represented by SEQ ID NO: 22, or a partial peptide thereof; etc., and (c) radiolabeled or fluorescence-labeled palmitic acid or myristic acid are reacted in the presence of a test compound;
[19b] A method for screening a prophylactic/therapeutic pharmaceutical agent for cancer, which comprises measuring and comparing the radioactivity or the fluorescent intensity derived from the palmitic acid or the myristic acid added to a peptide having an amino acid sequence comprised of the 24th to the 197th amino acids of the amino acid sequence represented by SEQ ID NO: 22, or a partial peptide thereof, in the case where (i) (a) the protein comprising the same or substantially the same amino acid sequence as the amino acid sequence represented by SEQ ID NO: 1, its partial peptide, or a salt thereof, (b) the peptide having an amino acid sequence comprised of the 24th to the 197th amino acids of the amino acid sequence represented by SEQ ID NO: 22, or a partial peptide thereof; etc., and (c) radiolabeled or fluorescence-labeled palmitic acid or myristic acid are reacted, and (ii) (a) the protein comprising the same or substantially the same amino acid sequence as the amino acid sequence represented by SEQ ID NO: 1, its partial peptide, or a salt thereof, (b) the peptide having an amino acid sequence comprised of the 24th to the 197th amino acids of the amino acid sequence represented by SEQ ID NO: 22, or a partial peptide thereof; etc., and (c) radiolabeled or fluorescence-labeled palmitic acid or myristic acid are reacted in the presence of a test compound;
[20] A method for preventing/treating cancer, which comprises inhibiting an activity of a protein comprising the same or substantially the same amino acid sequence as the amino acid sequence represented by SEQ ID NO: 1, its partial peptide, or a salt thereof, or inhibiting the expression of a gene for the protein, its partial peptide, or a salt thereof;
[21] A method for preventing/treating cancer, which comprises administering to a mammal an effective dose of (i) a compound or its salt that inhibits an activity of a protein comprising the same or substantially the same amino acid sequence as the amino acid sequence represented by SEQ ID NO: 1, its partial peptide, or a salt thereof, (ii) a compound or its salt that inhibits the expression of a gene for the protein, its partial peptide, or a salt thereof, (iii) the antibody according to [8], or (iv) an antisense polynucleotide comprising the entirety or part of a nucleotide sequence complementary or substantially complementary to a nucleotide sequence of a polynucleotide encoding the protein or its partial peptide;
[22] Use of (i) a compound or its salt that inhibits an activity of a protein comprising the same or substantially the same amino acid sequence as the amino acid sequence represented by SEQ ID NO: 1, its partial peptide, or a salt thereof, (ii) a compound or its salt that inhibits the expression of a gene for the protein, its partial peptide, or a salt thereof, (iii) the antibody according to [8], or (iv) an antisense polynucleotide comprising the entirety or part of a nucleotide sequence complementary or substantially complementary to a nucleotide sequence of a polynucleotide encoding the protein or its partial peptide for the manufacture of a prophylactic/therapeutic agent for cancer,;
[23] An agent for promoting apoptosis of cancer cells or suppressing proliferation of cancer cells, comprising a compound or its salt that inhibits an activity of a protein comprising the same or substantially the same amino acid sequence as the amino acid sequence represented by SEQ ID NO: 1, its partial peptide, or a salt thereof;
[24] An agent for promoting apoptosis of cancer cells or suppressing proliferation of cancer cells, comprising a compound or its salt that inhibits the expression of a gene for a protein comprising the same or substantially the same amino acid sequence as the amino acid sequence represented by SEQ ID NO: 1, its partial peptide, or a salt thereof;
[25] The pharmaceutical agent according to [4], which is an agent for promoting apoptosis of cancer cells or suppressing proliferation of cancer cells;
[26] The pharmaceutical agent according to [6], which is an agent for promoting apoptosis of cancer cells or suppressing proliferation of cancer cells;
[27] An agent for promoting apoptosis of cancer cells or suppressing proliferation of cancer cells, comprising the antibody according to [8];
[28] A method for screening a pharmaceutical agent for promoting apoptosis of cancer cells or suppressing proliferation of cancer cells, which comprises using a protein comprising the same or substantially the same amino acid sequence as the amino acid sequence represented by SEQ ID NO: 1, its partial peptide, or a salt thereof;
[29] A kit for screening a pharmaceutical agent for promoting apoptosis of cancer cells or suppressing proliferation of cancer cells, comprising a protein comprising the same or substantially the same amino acid sequence as the amino acid sequence represented by SEQ ID NO: 1, its partial peptide, or a salt thereof;
[30] A method for screening a pharmaceutical agent for promoting apoptosis of cancer cells or suppressing proliferation of cancer cells, which comprises using a polynucleotide encoding a protein comprising the same or substantially the same amino acid sequence as the amino acid sequence represented by SEQ ID NO: 1, or its partial peptide;
[31] A kit for screening a pharmaceutical agent for promoting apoptosis of cancer cells or suppressing proliferation of cancer cells, comprising a polynucleotide encoding a protein comprising the same or substantially the same amino acid sequence as the amino acid sequence represented by SEQ ID NO: 1, or its partial peptide;
[32] A method for promoting apoptosis of cancer cells or suppressing proliferation of cancer cells, which comprises inhibiting an activity of a protein comprising the same or substantially the same amino acid sequence as the amino acid sequence represented by SEQ ID NO: 1, its partial peptide, or a salt thereof, or inhibiting the expression of a gene for the protein, its partial peptide, or a salt thereof;
[33] A method for promoting apoptosis of cancer cells or suppressing proliferation of cancer cells, which comprises administering to a mammal an effective dose of (i) a compound or its salt that inhibits an activity of a protein comprising the same or substantially the same amino acid sequence as the amino acid sequence represented by SEQ ID NO: 1, its partial peptide, or a salt thereof, (ii) a compound or its salt that inhibits the expression of a gene for the protein, its partial peptide or a salt thereof, (iii) an antibody against the protein, its partial peptide, or a salt thereof, or (iv) an antisense polynucleotide comprising the entirety or part of a nucleotide sequence complementary or substantially complementary to a nucleotide sequence of a polynucleotide encoding the protein or its partial peptide;
[34] Use of (i) a compound or its salt that inhibits an activity of a protein comprising the same or substantially the same amino acid sequence as the amino acid sequence represented by SEQ ID NO: 1, its partial peptide, or a salt thereof, (ii) a compound or its salt that inhibits the expression of a gene for the protein, its partial peptide, or a salt thereof, (iii) an antibody against the protein, its partial peptide, or a salt thereof, or (iv) an antisense polynucleotide comprising the entirety or part of a nucleotide sequence complementary or substantially complementary to a nucleotide sequence of a polynucleotide encoding the protein or its partial peptide for the manufacture of a medicament for promoting apoptosis of cancer cells or suppressing proliferation of cancer cells; and the like.

The "prophylactic/therapeutic agent for cancer" may be a substance itself having a prophylactic/therapeutic action on cancer (e.g., synthetic compounds, peptides, proteins, antibodies, non-peptide compounds, fermentation products, cell extracts, plant extracts, animal tissue extracts, plasma, etc.) or a pharmaceutical agent containing such a substance.

The "agent for promoting apoptosis of cancer cells" may be a substance itself having an apoptosis promoting action on cancer cells, or a pharmaceutical agent containing such a substance (e.g., synthetic compounds, peptides, proteins, antibodies, non-peptide compounds, fermentation products, cell extracts, plant extracts, animal tissue extracts, plasma, etc.).

The "agent for suppressing proliferation of cancer cells" may be a substance itself having a proliferation suppressing action on cancer cells (e.g., synthetic compounds, peptides, proteins, antibodies, non-peptide compounds, fermentation products, cell extracts, plant extracts, animal tissue extracts, plasma, etc.), or a pharmaceutical agent containing such a substance.

### BRIEF DESCRIPTION OF THE DRAWINGS

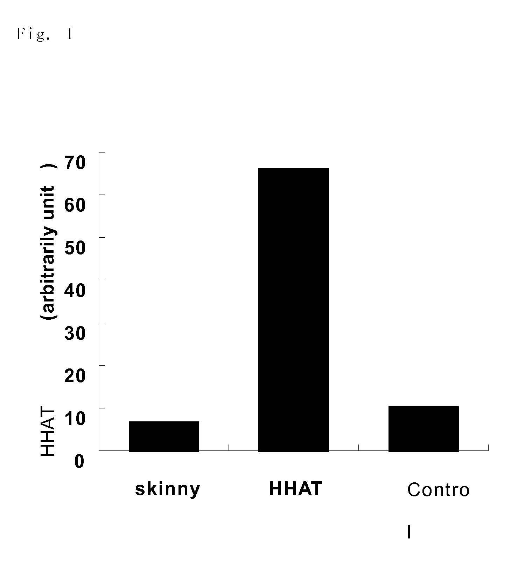

Fig. 1 shows the results of quantification of HHAT activity represented with the radioactivity of [¹⁴C]-palmitic acid added to human Shh-N. In the figure, the vertical axis represents the HHAT activity, and the horizontal axis represents the name of the genes expressed in the HEK293 cells.

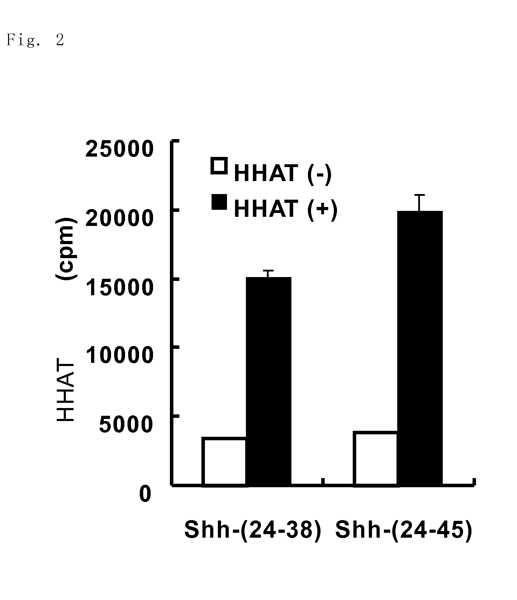

Fig. 2 shows the results of quantification of HHAT activity by means of the radioactivity of [³H]-palmitic acid added to the substrate peptide Shh-(24-38) or Shh-(24-45) when a reaction was carried out for 1 hour using 10 µl of each peptide using the activity measurement method described herein (n = 3). In the figure, the vertical axis represents the HHAT activity shown with the radioactivity (cpm), and the horizontal axis represents the substrate peptides used. HHAT(-) shows the values of the control experiment in which the HHAT enzyme solution was not used.

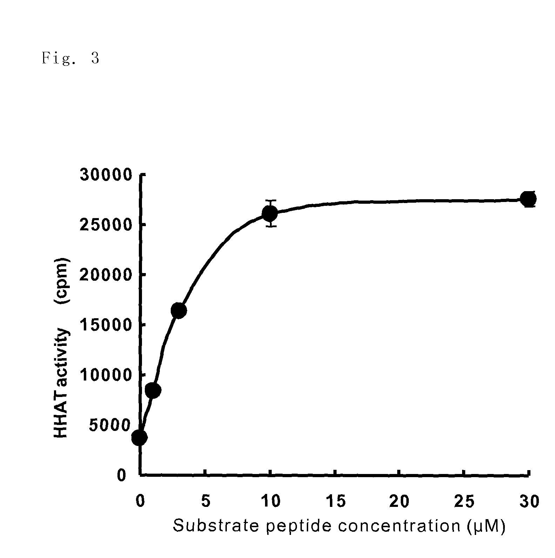

Fig. 3 shows the results of quantification of HHAT activity by means of the radioactivity of [³H]-palmitic acid added to Shh-(24-45) (n = 3). In the figure, the vertical axis represents the HHAT activity shown with the radioactivity (cpm), and the horizontal axis represents the amount of the substrate peptide (µM).

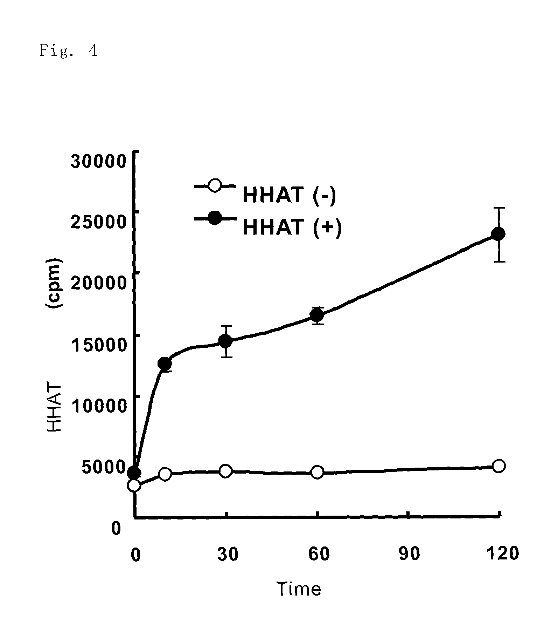

Fig. 4 shows the results of quantification of HHAT activity by means of the radioactivity of [³H]-palmitic acid added to Shh-(24-45) (n = 3). In the figure, the vertical axis represents the HHAT activity shown with the radioactivity (cpm), and the horizontal axis represents the reaction time (minutes). HHAT(-) shows the values of the control experiment in which the HHAT enzyme solution was not used.

### BEST MODE FOR CARRYING OUT THE INVENTION

The protein, which has the same or substantially the same amino acid sequence as the amino acid sequence represented by SEQ ID NO: 1 (hereinafter, the protein will be sometimes referred to as the "protein of the present invention" or the "protein used in the present invention") may be any protein derived from any cells of human and warm-blooded animals (e.g., guinea pig, rat, mouse, fowl, rabbit, swine, ovine, bovine, simian, etc.) (e.g., retina cells, hepatocyte, splenocyte, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes, platelets, etc.), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); any tissue where such a cell is present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine), blood vessel, heart, thymus, spleen, submandibular gland, peripheral blood, prostate, testicle, ovary, placenta, uterus, bone, joint, skeletal muscle, etc.); or any blood cell-type cell or a cultured cell thereof (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); and the protein may also be a synthetic protein.

The amino acid sequence having substantially the same amino acid sequence as that represented by SEQ ID NO: 1 encompasses amino acid sequences having at least about 50% homology, preferably at least about 60% homology, more preferably at least about 70% homology, much more preferably at least about 80% homology, further much more preferably at least about 90% homology and most preferably at least about 95% homology, to the amino acid sequence shown by SEQ ID NO: 1; and so on.

Homology of the amino acid sequences can be determined under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

Preferred examples of the protein comprising substantially the same amino acid sequence as the amino acid sequence represented by SEQ ID NO: 1 include proteins having substantially the same amino acid sequence as the amino acid sequence represented by SEQ ID NO: 1 and having an activity of substantially the same property as that of the protein comprising the amino acid sequence represented by SEQ ID NO: 1, and the like.

The substantially equivalent activity encompasses, for example, an acyltransferase activity, and the like. The term "substantially equivalent" is used to mean that the activities are the same in nature (e.g., physiologically or pharmacologically). Thus, although it is preferred that the acyltransferase activities are equivalent (e.g., about 0.01 to 100 times, preferably about 0.1 to 10 times, more preferably 0.5 to 2 times), quantitative factors such as a level of these activities, a molecular weight of the protein, etc. may differ.

The acyltransferase activity can be measured by any known method, e.g., the method described in Genes Dev., 18, 641-659, 2004, or a method conformed thereto. Specifically, (a) the protein of the present invention, (b) a peptide at the N-terminus of Shh (e.g., human Shh, mouse Shh, etc.) (e.g., a peptide, or a partial peptide thereof, having an amino acid sequence comprised of the 24th to the 197th amino acids of the amino acid sequence represented by SEQ ID NO: 22; etc.), and (c) palmitic acid which is radiolabeled (e.g., labeled with [¹²⁵I], [¹³¹I], [³H], [¹⁴C], [³²P], [³³P], [³⁵S], etc.) or fluorescence-labeled (e.g., labeled with a cyanine fluorescence dye (e.g., Cy2, Cy3, Cy5, Cy5.5, Cy7 (manufactured by Amersham Biosciences) etc.) are reacted, and the radioactivity or the fluorescent intensity derived from the palmitic acid added to the N-terminal peptide of Shh is measured. The reaction is carried out in an appropriate buffer. After the enzyme reaction, the N-terminal peptide of Shh is separated by, for example, SDS-polyacrylamide gel electrophoresis. The radioactivity or the fluorescent intensity is measured by any known method using a scintillation counter, fluorography or the like. Instead of palmitic acid, myristic acid may be used.

Examples of the protein used in the present invention include so-called muteins such as proteins comprising (i) an amino acid sequence resulting from deletions of at least 1 or 2 (e.g., about 1 to about 100, preferably about 1 to about 30, more preferably about 1 to about 10 and still more preferably several (1 to 5)) amino acids in the amino acid sequence represented by SEQ ID NO: 1; (ii) an amino acid sequence resulting from additions of at least 1 or 2 (e.g., about 1 to about 100, preferably about 1 to about 30, more preferably about 1 to about 10 and still more preferably several (1 to 5)) amino acids to the amino acid sequence represented by SEQ ID NO: 1; (iii) an amino acid sequence resulting from insertions of at least 1 or 2 (e.g., about 1 to about 100, preferably about 1 to about 30, more preferably about 1 to about 10 and still more preferably several (1 to 5)) amino acids into the amino acid sequence represented by SEQ ID NO: 1; (iv) an amino acid sequence resulting from substitutions of at least 1 or 2 (e.g., about 1 to about 100, preferably about 1 to about 30, more preferably about 1 to about 10 and still more preferably several (1 to 5)) amino acids by other amino acids in the amino acid sequence represented by SEQ ID NO: 1; or (v) a combination of any of these amino acid sequences.

Where the amino acid sequence is inserted, deleted or substituted as described above, the position of its insertion, deletion or substitution is not particularly limited.

Throughout the specification, the proteins are represented in accordance with the conventional way of describing peptides, that is, with the N-terminus (amino terminus) being at the left hand and the C-terminus (carboxyl terminus) being at the right hand. In the protein used in the present invention including the protein comprising the amino acid sequence represented by SEQ ID NO: 1, the C-terminus may be in any form of a carboxyl group (-COOH), a carboxylate (-COO-), an amide (-CONH₂) and an ester (-COOR).

Herein, examples of R of the ester group include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂ alkyl group, e.g., benzyl, phenethyl, etc., or an α-naphthyl-C₁₋₂ alkyl group such as α-naphthylmethyl, etc.; a pivaloyloxymethyl group; and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include those wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains; etc.

Specific examples of the protein used in the present invention include a protein comprising the amino acid sequence represented by SEQ ID NO: 1, a protein comprising the amino acid sequence represented by SEQ ID NO: 3, a protein comprising the amino acid sequence represented by SEQ ID NO: 5, and the like.

The partial peptide of the protein used in the present invention may be any peptide as long as it is a partial peptide of the protein used in the present invention described above and preferably has the property equivalent to that of the protein used in the present invention described above.

For the purpose of preparing an antibody of the present invention described below, specific examples of the peptide include a peptide having the 27th up to the 40th amino acids or the 156th up to the 169th amino acids of the amino acid sequence represented by SEQ ID NO: 1. Preferably used are peptides having, e.g., at least 20, preferably at least 50, more preferably at least 70, much more preferably at least 100, and most preferably at least 200 amino acids in the constituent amino acid sequence of the protein used in the present invention, and the like.

The partial peptide used in the present invention may contain amino acid sequences, of which at least 1 or 2 amino acids (preferably about 1 to about 20 amino acids, more preferably about 1 to about 10 amino acids and still more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added; or, in which at least 1 or 2 amino acids (preferably about 1 to about 20 amino acids, more preferably about 1 to about 10 amino acids and still more preferably several (1 to 5) amino acids) are substituted by other amino acids.

In the partial peptide used in the present invention, the C-terminus may be in any form of a carboxyl group (-COOH), a carboxylate (-COO-), an amide (-CONH₂) or an ester (-COOR).

Furthermore, examples of the partial peptide used in the present invention include those having a carboxyl group (or a carboxylate) at a position other than the C-terminus, those having an amino group protected with a protecting group at the N-terminal amino acid residues (e.g., methionine residue); those being cleaved at the N-terminal region in vivo and with the glutamyl group thus formed being pyroglutaminated; those having a substituent on the side chain of an amino acid in the molecule wherein the substituent is protected with a suitable protecting group, or conjugated peptides such as so-called glycopeptides having sugar chains; etc., as in the protein used in the present invention described above.

The partial peptide used in the present invention may also be used as an antigen for producing antibodies.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), and the like.

The protein, its partial peptide, or a salt thereof used in the present invention may be manufactured by known methods used to purify a protein from human or warm-blooded animal cells or tissues described above. Alternatively, they may also be manufactured by culturing transformants containing DNAs encoding these proteins. Furthermore, they may also be manufactured by any method conformed to a peptide synthesis method described later.

For manufacturing from human or mammalian tissues or cells, human or mammalian tissues or cells are homogenized, extracted with an acid or the like, and the extract is purified/isolated by a combination of chromatography techniques such as reverse phase chromatography, ion exchange chromatography, and the like.

To synthesize the protein, its partial peptide, a salt thereof, or an amide thereof used in the present invention, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2', 4'-dimethoxyphenyl-hydroxymethyl)phenoxy resin, 4-(2', 4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, any of a variety of activation reagents usable for protein synthesis may be used. Carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) may be added directly to the resin, or the protected amino acids may be previously activated in the form of symmetric acid anhydrides, HOBt esters or HOOBt esters, followed by addition of the activated protected amino acids to the resin.

Solvents suitable for use to activate the protected amino acids or condense the protected amino acids with the resin may be appropriately selected from solvents known to be usable for protein condensation reactions. Examples of such solvents include acid amides such as N,N-dimethylformamide, N,N-dimethylacetamide, N-methylpyrrolidone, etc.; halogenated hydrocarbons such as methylene chloride, chloroform, etc.; alcohol such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; appropriate mixtures of any of these solvents; and the like. The reaction temperature is appropriately selected from the range known to be applicable to protein binding reactions and is usually selected from the range of approximately -20°C to 50°C. The activated amino acid derivatives are generally used in an excess of 1.5 to 4 times. The condensation is examined by a test using the ninhydrin reaction. When the condensation is found to be insufficient as a result of the test, the condensation can be completed by repeating the condensation reaction without removal of the protecting groups. When the condensation is still insufficient even after repeating the reaction, unreacted amino acids can be acetylated with acetic anhydride or acetylimidazole, so that the future reactions will not be influenced.

Examples of the protecting group used to protect the amino groups in the starting materials include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, for example, alkyl esterification (e.g., esterification in the form of linear, branched or cyclic alkyl esters of methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester), phenacyl esterification, benzyloxycarbonyl hydrazidation, t-butoxycarbonyl hydrazidation, trityl hydrazidation, or the like.

A hydroxyl group of serine can be protected through, for example, esterification or etherification. Examples of groups appropriately used for esterification include a lower (C₁₋₆) alkanoyl group such as acetyl group, etc., an aroyl group such as benzoyl group, etc., and a group derived from carbonic acid such as benzyloxycarbonyl group, ethoxycarbonyl group, etc. Examples of groups appropriately used for etherification include benzyl group, tetrahydropyranyl group, t-butyl group, etc.

Examples of groups used to protect a phenolic hydroxyl group of tyrosine include Bzl, Cl₂-Bzl, 2-nitrobenzyl, Br-Z, t-butyl, etc.

Examples of groups used to protect an imidazole moiety of histidine include Tos, 4-methoxy-2,3,6-trimethylbenzenesulfonyl, DNP, benzyloxymethyl, Bum, Boc, Trt, Fmoc, etc.

Examples of the activated form of the carboxyl group in the starting materials include the corresponding acid anhydride, azides, activated esters (esters with alcohols (e.g., pentachlorophenol, 2,4,5-trichlorophenol, 2,4-dinitrophenol, cyanomethyl alcohol, paranitrophenol, HONB, N-hydroxysuccimide, N-hydroxyphthalimide, HOBt)), etc. Examples of the activated form of the amino acids in the starting materials include the corresponding phosphoric amides.

Examples of a method usable for eliminating (splitting off) the protecting group include catalytic reduction under hydrogen gas flow in the presence of a catalyst such as Pd-black, Pd-carbon, etc.; acid treatment with anhydrous hydrogen fluoride, methanesulfonic acid, trifluoromethanesulfonic acid or trifluoroacetic acid, or a mixture solution thereof; base treatment with diisopropylethylamine, triethylamine, piperidine, piperazine, etc.; reduction with sodium in liquid ammonia; and the like. The elimination of the protecting group by the acid treatment described above is carried out generally at a temperature of approximately -20 to 40°C. In the acid treatment, it is effective to add a cation scavenger such as anisole, phenol, thioanisole, metacresol, paracresol, dimethylsulfide, 1,4-butanedithiol, 1,2-ethanedithiol, etc. Furthermore, 2,4-dinitrophenyl group known as the protecting group for the imidazole of histidine is removed by thiophenol treatment. Formyl group used as the protecting group of the indole of tryptophan is eliminated by deprotection caused by the acid treatment in the presence of 1,2-ethanedithiol, 1,4-butanedithiol, etc. as well as by alkali treatment with a dilute sodium hydroxide solution, dilute ammonia, etc.

Protection of the functional groups that should not be involved in the reaction of the starting materials, protecting groups usable for such protection, elimination of the protecting groups, activation of the functional groups involved in the reaction, or the like may be appropriately selected from known groups or known means.

According to another exemplary method, the amide of the protein or partial peptide is obtained as follows. An α-carboxyl group of the carboxy terminal amino acid is first protected by amidation, and then the peptide (protein) chain is extended from the amino group side to a desired length. Thereafter, a protein or partial peptide obtained by eliminating only the protecting group of the N-terminal α-amino group from the polypeptide, and a protein or partial peptide obtained by eliminating only the protecting group of the C-terminal carboxyl group, are produced. The proteins or partial peptides are condensed in a mixture of the solvents described above. The details of the condensation reaction are substantially the same as described above. After the protected protein or peptide obtained by the condensation is purified, all the protecting groups are eliminated by the above-described method to give a desired crude protein or peptide. This crude protein or peptide is purified by any of various known purification means, and the major fraction is lyophilized. Thus, an amidated form of a desired protein or peptide is obtained.

An esterified form of the protein or peptide may be obtained as follows, for example. An α-carboxyl group of the carboxy terminal amino acid is condensed with a desired alcohol to prepare the amino acid ester. After this, an esterified form of a desired protein or peptide can be obtained by substantially the same procedure as for the amidated form of the desired protein or peptide.

The partial protein or a salt thereof used in the present invention can be produced by any known method for peptide synthesis, or by cleaving the protein used in the present invention with an appropriate peptidase. The method for peptide synthesis may be, for example, either solid phase synthesis or liquid phase synthesis. That is, the partial peptide or amino acids that can construct the partial peptide used in the present invention is condensed with the remaining part. Where the product contains protecting groups, these protecting groups are eliminated to give a desired peptide. Publicly known methods for condensation and elimination of the protecting groups are described in (i) - (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co., Ltd. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (Development of Pharmaceuticals, Sequel), Vol. 14, Peptide Synthesis, published by Hirokawa Shoten

After the reaction, the product can be purified by a combination of usual purification methods such as solvent extraction, distillation, column chromatography, liquid chromatography, recrystallization and the like to isolate the partial peptide used in the present invention. When the partial peptide obtained by any of the above methods is in a free form, the partial peptide can be converted into an appropriate salt by a known method or a method conformed thereto. Conversely, when obtained in a salt form, the partial peptide can be converted into a free form by a known method or a method conformed thereto.

The polynucleotide encoding the protein used in the present invention may be any polynucleotide which has the nucleotide sequence encoding the protein used in the present invention described above. A DNA is preferable, and such a DNA may be any of genomic DNA, genomic DNA library, cDNA derived from the cells and tissues described above, cDNA library derived from the cells and tissues described above, and synthetic DNA.

The vector to be used for the library may be any of bacteriophage, plasmid, cosmid, phagemid and the like. The vector may also be directly amplified by reverse transcriptase polymerase chain reaction (hereinafter, abbreviated as "RT-PCR") using total RNA or mRNA fraction prepared from the cells and tissues described above.

The DNA encoding the protein used in the present invention may be, for example, any DNA containing a nucleotide sequence represented by SEQ ID NO: 2 or any DNA containing a nucleotide sequence hybridizable to the nucleotide sequence represented by SEQ ID NO: 2 under high stringent conditions and encoding a protein which has activities substantially equivalent to those of the protein comprising the amino acid sequence represented by SEQ ID NO: 1.

The DNA hybridizable to the nucleotide sequence represented by SEQ ID NO: 2 under high stringent conditions may be, for example, a DNA containing a nucleotide sequence having at least about 50% homology, preferably at least about 60% homology, more preferably at least about 70% homology, still more preferably at least about 80% homology, especially preferably at least about 90%, and most preferably at least about 95% to the nucleotide sequence represented by SEQ ID NO: 2.

Homology of nucleotide sequences can be determined under the following conditions (an expectation value = 10; gaps are allowed; filtering = ON; matching score = 1; mismatching score = -3) using a homology scoring algorithm NCBI BLAST (National Center for Biotechnology Information Basic Local Alignment Search Tool).

The hybridization can be carried out by any known method or a method conformed thereto, for example, a method described in Molecular Cloning, 2nd (J. Sambrook et al., Cold Spring Harbor Lab. Press, 1989). The hybridization may also be performed using a commercially available library in accordance with the protocol described in the attached instructions. Preferably, the hybridization may be carried out under high stringent conditions.

The "high stringent conditions" used herein refers to, for example, the conditions of a sodium concentration at about 19 to about 40 mM, preferably about 19 to about 20 mM at a temperature of about 50 to about 70°C, preferably about 60 to about 65°C. In particular, the conditions of a sodium concentration of about 19 mM at a temperature of about 65°C are most preferred.

More specifically, a DNA encoding a protein containing the amino acid sequence represented by SEQ ID NO: 1 may be a DNA containing the nucleotide sequence represented by SEQ ID NO: 2 or the like; a DNA encoding a protein containing the amino acid sequence represented by SEQ ID NO: 3 may be a DNA containing the nucleotide sequence represented by SEQ ID NO: 4 or the like; a DNA encoding a protein containing the amino acid sequence represented by SEQ ID NO: 5 may be a DNA containing the nucleotide sequence represented by SEQ ID NO: 6 or the like.

The DNA encoding the partial peptide used in the present invention may be any DNA which contains a nucleotide sequence encoding the above-mentioned partial peptide used in the present invention. Such a DNA may be any of genomic DNA, genomic DNA library, cDNA derived from the cells and tissues described above, cDNA library derived from the cells and tissues described above, and synthetic DNA.

The DNA encoding the partial peptide used in the present invention may be, for example, a DNA containing a part of the DNA having the nucleotide sequence represented by SEQ ID NO: 2, or a DNA containing a nucleotide sequence hybridizable to the nucleotide sequence represented by SEQ ID NO: 2 under stringent conditions and containing a part of the DNA encoding a protein having an activity substantially equivalent to that of the protein of the present invention.

The "DNA hybridizable to the nucleotide sequence represented by SEQ ID NO: 2" has the same significance as described above.

The hybridization methods and high stringent conditions may be substantially the same as described above.

The DNA completely encoding a protein or a partial peptide used in the present invention (hereinafter, these will be sometimes referred to simply as the "protein of the present invention" in the explanation of the cloning and expression of a DNA encoding such a protein or partial peptide) may be cloned as follows. The DNA may be amplified by a known PCR method using a synthetic DNA primer containing a part of the nucleotide sequence encoding the protein of the present invention. Alternatively, the DNA inserted into an appropriate vector may be selected by hybridization with a DNA fragment encoding a partial or entire area of the protein of the present invention or with a labeled DNA fragment. The hybridization can be carried out, for example, in accordance with a method described in Molecular Cloning, 2nd, (J. Sambrook et al., Cold Spring Harbor Lab. Press, 1989). The hybridization may also be performed using a commercially available library in accordance with the protocol described in the attached instructions.

Conversion of the nucleotide sequence of the DNA can be effected by a known method such as the ODA-LA PCR method, the Gapped duplex method, the Kunkel method, etc. or a method conformed thereto, using a known kit, for example, Mutan^{™}-superExpress Km (Takara Shuzo Co., Ltd.), Mutan^{™}-K (Takara Shuzo Co., Ltd.), etc.

The cloned DNA encoding the protein can be used as it is, or if desired, after digestion with a restriction enzyme or after addition of a linker thereto, depending upon the purpose. The DNA may contain ATG as a translation initiation codon at the 5' terminus thereof and may further contain TAA, TGA or TAG as a translation termination codon at the 3' terminus thereof. These translation initiation and termination codons may also be added by using an appropriate synthetic DNA adapter.

An expression vector for the protein of the present invention can be produced by, for example, (i) excising a desired DNA fragment from the DNA encoding the protein of the present invention, and then (ii) ligating the DNA fragment with an appropriate expression vector downstream with respect to the promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter which matches well with a host to be used for gene expression. In the case where animal cells are used as the host, examples of the promoter include SRα promoter, SV40 promoter, LTR promoter, CMV promoter, HSV-TK promoter, etc.

Among them, CMV (cytomegalovirus) promoter, SRα promoter and the like are preferably used. Where the host is bacteria belonging to the genus Escherichia, preferable examples of the promoter include trp promoter, lac promoter, recA promoter, λP_{L} promoter, Ipp promoter, T7 promoter, etc. Where the host is bacteria belonging to the genus Bacillus, preferable examples of the promoter include SPO1 promoter, SPO2 promoter, penP promoter, etc. Where the host is yeast, preferable examples of the promoter include PHO5 promoter, PGK promoter, GAP promoter, ADH promoter, etc. Where the host is insect cells, preferable examples of the promoter include polyhedrin prompter, P10 promoter, etc.

In addition to the foregoing examples, substances containing an enhancer, a splicing signal, a polyA addition signal, a selection marker, any SV40 replication origin (hereinafter, occasionally abbreviated as "SV40ori"), etc. may be used as the expression vector. Examples of the selection marker include dihydrofolate reductase (hereinafter, occasionally abbreviated as "dhfr") gene [methotrexate (MTX) resistance], ampicillin resistant gene (hereinafter, occasionally abbreviated as "Amp^{r}"), neomycin resistant gene (hereinafter, occasionally abbreviated as "Neo^{r}", G418 resistance), etc. In particular, when dhfr gene is used as the selection marker in dhfr gene deficient Chinese hamster cells, the objective gene may be selected on a thymidine-free media.

When necessary, a signal sequence that matches with the host is added to the N-terminus of the protein of the present invention. Where the host is bacteria belonging to the genus Escherichia, PhoA signal sequence, OmpA signal sequence, etc. are usable as the signal sequence. Where the host is bacteria belonging to the genus Bacillus, α-amylase signal sequence, subtilisin signal sequence, etc. are usable as the signal sequence. Where the host is yeast, MFα signal sequence, SUC2 signal sequence, etc. are usable as the signal sequence. Where the host is animal cells, insulin signal sequence, α-interferon signal sequence, antibody molecule signal sequence, etc. are usable as the signal sequence.

Using the vector containing the DNA encoding the protein of the present invention thus constructed, a transformant can be produced.

Examples of the host include bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)), JM103 (Nucleic Acids Research, 9, 309 (1981)], JA221 [Journal of Molecular Biology, 120, 517 (1978)], HB101 [Journal of Molecular Biology, 41, 459 (1969)), C600 (Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of the yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of the insect cells are as follows. For the virus AcNPV, Spodoptera frugiperda cells (Sf cells), MG1 cells derived from mid-intestine of Trichoplusia ni, High Five^{™} cells derived from egg of Trichoplusia ni, cells derived from Mamestra brassicae, cells derived from Estigmena acrea, etc. are usable. For the virus BmNPV, Bombyx mori N cells (BmN cells), etc. are usable. Examples of the Sf cell include Sf9 cells (ATCC CRL1711), Sf21 cells (both cells are described in Vaughn, J. L. et al., In Vivo, 13, 213-217 (1977)),etc.

As the insect, for example, a larva of Bombyx mori, and the like are usable [Maeda, et al., Nature, 315, 592 (1985)].

Examples of the animal cells include monkey cells COS-7 (COS7), Vero, Chinese hamster cells CHO (hereinafter, abbreviated as "CHO cells"), dhfr gene deficient Chinese hamster cells CHO (hereinafter, abbreviated as "CHO (dhfr⁻) cells"), mouse L cells, mouse AtT-20, mouse myeloma cells, mouse ATDC5 cells, rat GH3, human FL cells, etc.

The bacteria of the genus Escherichia can be transformed in accordance with, for example, a method described in Proc. Natl. Acad. Sci. U.S.A., 69, 2110 (1972), Gene, 17, 107 (1982), etc.

The bacteria of the genus Bacillus can be transformed in accordance with, for example, a method described in Molecular & General Genetics, 168, 111 (1979), etc.

Yeast can be transformed in accordance with, for example, a method described in Methods in Enzymology, 194, 182-187 (1991), Proc. Natl. Acad. Sci. U.S.A., 75, 1929 (1978), etc.

Insect cells or insects can be transformed in accordance with, for example, a method described in Bio/Technology, 6, 47-55(1988), etc.

Animal cells can be transformed in accordance with, for example, a method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, a transformant transformed with an expression vector containing the DNA encoding the protein of the present invention can be obtained.

For culturing a transformant having bacteria belonging to the genus Escherichia or the genus Bacillus as the host, a liquid medium is appropriately usable. The liquid medium is supplemented with materials required for growth of the transformant such as carbon sources, nitrogen sources, inorganic materials, and the like. Examples of the carbon sources include glucose, dextrin, soluble starch, sucrose, etc. Examples of the nitrogen sources include inorganic or organic materials such as ammonium salts, nitrates, corn steep liquor, peptone, casein, meat extract, soybean cake, potato extract, etc. Examples of the inorganic materials include calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferable example of the medium for incubation of the bacteria belonging to the genus Escherichia is M9 medium supplemented with glucose and Casamino acid (Miller, Journal of Experiments in Molecular Genetics, 431-433, Cold Spring Harbor Laboratory, New York, 1972). When necessary, a chemical such as 3β-indolylacrylic acid or the like may be added to the medium to make the promoter act efficiently.

Where the host is bacteria belonging to the genus Escherichia, the transformant is usually cultivated at about 15 to about 43°C for about 3 to about 24 hours. When necessary, the culture may be aerated or agitated.

Where the host is bacteria belonging to the genus Bacillus, the transformant is usually cultivated at about 30 to about 40°C for about 6 to about 24 hours. When necessary, the culture may be aerated or agitated.

Where the host is yeast, the transformant is cultivated in, for example, Burkholder's minimal medium [Bostian, K. L. et al., Proc. Natl. Acad. Sci. U.S.A., 77, 4505 (1980)] or SD medium supplemented with 0.5% Casamino acid [Bitter, G. A. et al., Proc. Natl. Acad. Sci. U.S.A., 81, 5330 (1984)]. Preferably, pH of the medium is adjusted to about 5 to about 8. The transformant is usually cultivated at about 20°C to about 35°C for about 24 to about 72 hours. When necessary, the culture may be aerated or agitated.

Where the host is insect cells or insects, the transformant is cultivated in, for example, Grace's Insect Medium (Grace, T. C. C., Nature, 195, 788 (1962)) to appropriately supplemented with an additive such as immobilized 10% bovine serum or the like. Preferably, pH of the medium is adjusted to about 6.2 to about 6.4. The transformant is usually cultivated at about 27°C for about 3 to 5 days. When necessary, the culture may be aerated or agitated.

Where the host is animal cells, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours. When necessary, the culture may be aerated or agitated.

As described above, the protein of the present invention can be produced into the cell, on the cell membrane, or out of the cell of the transformant.

The protein of the present invention can be separated by purification of any of the cultures described above by the following procedure.

The protein of the present invention can be extracted from the culture or cells as follows. After cultivation, the transformants or cells are collected by a known method and suspended in an appropriate buffer solution. The transformants or cells are then disrupted by ultrasonication, treatment with lysozyme and/or freeze-thaw cycling, followed by centrifugation, filtration, etc. Thus, a crude extract of polypeptide is obtained. The buffer solution may contain a protein denaturant such as urea, guanidine hydrochloride or the like, or a surfactant such as Triton X-100^{™}, or the like. When the protein is secreted in the culture fluid, after the completion of the cultivation, the supernatant can be separated and collected from the transformants or cells by a known method.

The protein contained in the culture supernatant or the extract thus obtained can be purified by appropriately combining known separation and purification methods. Examples of such known separation and purification methods include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

In the case where the protein thus obtained is in a free form, the protein can be converted into a salt by any known method or a method conformed thereto. By contrast, when the protein is in the form of a salt, the protein can be converted into a free form or a different salt by any known method or a method conformed thereto.

The protein produced by a recombinant can be treated, prior to or after the purification, with an appropriate protein modifying enzyme so that the protein can be arbitrarily modified or partially deprived of the polypeptide. Examples of the protein-modifying enzyme include trypsin, chymotrypsin, arginyl endopeptidase, protein kinase, glycosidase, and the like.

The presence of the protein thus produced can be measured by enzyme immunoassay or Western blotting using a specific antibody, for example.

An antibody to the protein, partial peptide or a salt thereof used in the present invention maybe any antibody which can recognize the protein, partial peptide or a salt thereof used in the present invention, and may be either a polyclonal antibody or a monoclonal antibody.

An antibody to the protein, partial peptide or a salt thereof used in the present invention (hereinafter, these will be sometimes referred to simply as the "protein of the present invention" in the explanation of the antibody) is produced by any known antibody or antiserum production method, using the protein of the present invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are simian, rabbit, canine, guinea pig, mouse, rat, ovine, goat and fowl, with the use of mouse and rat being preferred.

For the preparation of monoclonal antibody-producing cells, a warm-blooded animal, e.g., mouse, immunized with an antigen wherein the antibody titer is noted is selected, then spleen or lymph node is collected after 2 to 5 days from the final immunization, and antibody-producing cells contained therein are fused with myeloma cells from a homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out by any known method, for example, by the method of Koehler and Milstein [Nature, 256, 495, (1975)]. Examples of the fusion accelerator include polyethylene glycol (PEG), Sendai virus, etc., of which PEG is preferably employed.

Examples of the myeloma cells include those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme, or Protein A, and detecting the monoclonal antibody bound to the solid phase; and a method which comprises adding the supernatant of hybridoma to a solid phase adsorbed with an anti-immunoglobulin antibody or Protein A, adding the protein labeled with a radioactive substance, an enzyme, etc., and detecting the monoclonal antibody bound to the solid phase; and the like.

The monoclonal antibody can be screened according to any known method or a method conformed thereto. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at about 37°C, for about 5 days to about 3 weeks, preferably 1 to 2 weeks, normally under 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of polyclonal antibody]

The polyclonal antibody of the present invention can be manufactured by any known method or a method conformed thereto. For example, a warm-blooded animal is immunized with an immunogen (protein antigen) per se, or a complex of an immunogen and a carrier protein in an a manner similar to the method described above for the manufacture of monoclonal antibodies. The product containing the antibody against the protein of the present invention is collected from the immunized animal followed by separation and purification of the antibody.

In the complex of an immunogen and a carrier protein used to immunize a warm-blooded animal, the type of carrier protein and the mixing ratio of the carrier to hapten may be any type and in any ratio, as long as the antibody is efficiently produced to the hapten immunized by crosslinking to the carrier. For example, bovine serum albumin, bovine thyroglobulin, hemocyanin or the like is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling, for example.

The condensation product is administered to the warm-blooded animal either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood, of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed used in the separation and purification of monoclonal antibodies described above.

The antisense polynucleotide having a nucleotide sequence, or a part thereof, complementary or substantially complementary to the nucleotide sequence of a polynucleotide encoding the protein or partial peptide used in the present invention (e.g., DNA; hereinafter, such a DNA will be sometimes referred to as the "DNA of the present invention" in the description of antisense polynucleotide) can be any antisense polynucleotide, so long as it possesses a nucleotide sequence, or a part thereof, complementary or substantially complementary to the nucleotide sequence of the DNA of the present invention and capable of suppressing the expression of said DNA, but antisense DNA is preferred.

Examples of the nucleotide sequence substantially complementary to the DNA of the present invention may include a nucleotide sequence having at least about 70% homology, preferably at least about 80% homology, more preferably at least about 90% homology and most preferably at least about 95% homology, to the entirety or a part of nucleotide sequence complementary to the DNA of the present invention (i.e., complementary strand to the DNA of the present invention), and the like. Especially in the entire nucleotide sequence of the complementary strand to the DNA of the present invention, preferred are (i) an antisense polynucleotide having at least about 70% homology, preferably at least about 80% homology, more preferably at least about 90% homology and most preferably at least about 95% homology, to the complementary strand of the nucleotide sequence which encodes the N-terminal region of the protein of the present invention (e.g., the nucleotide sequence around the initiation codon) in the case of antisense polynucleotide directed to translation inhibition and (ii) an antisense polynucleotide having at least about 70% homology, preferably at least about 80% homology, more preferably at least about 90% homology and most preferably at least about 95% homology, to the complementary strand of the entire nucleotide sequence of the DNA of the present invention having intron, in the case of antisense polynucleotide directed to RNA degradation by RNaseH.

Specific examples include an antisense polynucleotide containing the entirety or part of a nucleotide sequence complementary or substantially complementary to a nucleotide sequence of DNA containing the nucleotide sequence represented by SEQ ID NO: 2 or SEQ ID NO: 4, preferably, for example, an antisense polynucleotide containing the entirety or part of a nucleotide sequence complementary to a nucleotide sequence of DNA containing the nucleotide sequence represented by SEQ ID NO: 2 or SEQ ID NO: 4 (more preferably, an antisense polynucleotide containing the entirety or part of a nucleotide sequence complementary to a nucleotide sequence of DNA containing the nucleotide sequence represented by SEQ ID NO: 2 or SEQ ID NO: 4), etc.

The antisense polynucleotide is generally constituted by nucleotides of about 10 to about 40, preferably about 15 to about 30.

To prevent digestion with a hydrolase such as nuclease, etc., the phosphoric acid residue (phosphate) of each nucleotide that constitutes the antisense DNA may be substituted with a chemically modified phosphoric acid residue, e.g., phosphorothioate, methyl phosphonate, phosphorodithionate, etc. Also, the sugar (deoxyribose) in each nucleotide may be substituted with a chemically modified structure such as 2'-O-methylation, etc. The base part (pyrimidine, purine) may also be chemically modified and may be any one which hybridizes to a DNA containing the nucleotide sequence represented by SEQ ID NO: 2. These antisense polynucleotides may be synthesized using a known DNA synthesizer, etc.

According to the present invention, the antisense polynucleotide (nucleic acid) corresponding to a gene for the protein of the present invention and capable of inhibiting the replication or expression of such a gene can be designed and synthesized based on the nucleotide sequence information of cloned or identified protein-encoding DNA. Such an antisense polynucleotide is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating and/or controlling the in vivo and ex vivo expression of the gene for the protein of the present invention, and are useful for the treatment or diagnosis of diseases, etc. The phrase "corresponding to" is used to mean homologous to or complementary to a particular sequence of the nucleotide including the gene, nucleotide sequence or nucleic acid. The term "corresponding" used regarding the relationship between nucleotides, nucleotide sequences or nucleic acids and proteins usually refers to amino acids of a protein under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, etc. may be selected as preferred target regions, though any other region in the protein genes may be selected as a target.

The relationship between the targeted nucleic acids and the polynucleotides complementary to at least a part of the target region, specifically in the case where the targeted nucleic acids are hybridizable to the target region, the targeted nucleic acid can be denoted to be "antisense" to the polynucleotide of the target region. Examples of the antisense polynucleotides include polynucleotides containing 2-deoxy-D-ribose, polynucleotides containing D-ribose, any other types of polynucleotides which are N-glycosides of a purine or pyrimidine base, other polymers having non-nucleotide backbones (e.g., commercially available protein nucleic acids and synthetic sequence-specific nucleic acid polymers), other polymers containing particular linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid include sulfur and thiophosphate derivatives of nucleic acids, those resistant to degradation of polynucleoside amides or oligonucleoside amides, etc. The antisense polynucleotide of the present invention can be modified, for example, based on the following design; that is, by increasing the intracellular stability of the antisense polynucleotide, enhancing the cell permeability of the antisense polynucleotide, increasing the affinity of the polynucleotide to the targeted sense strand to a higher level, or minimizing the toxicity, if any, of the antisense polynucleotide. Many of such modifications are known in the art, as disclosed in Pharm. Tech. Japan, Vol. 8, pp. 247, 1992; Vol. 8, pp. 395, 1992; Antisense Research and Applications, CRC Press, 1993; etc.

The antisense polynucleotide of the present invention may contain altered or modified sugars, bases or linkages. The antisense polynucleotide may also be provided in a specialized form such as liposomes or microspheres may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached include cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory activity of the antisense polynucleotide can be examined using the transformant of the present invention, the in vivo and ex vivo gene expression system of the present invention, or the in vivo and ex vivo translation system for the protein of the present invention.

Hereinafter, the protein of the present invention, its partial peptide, or a salt thereof (hereinafter, sometimes referred to simply as the "protein of the present invention"), a polynucleotide (e.g., DNA) (hereinafter, sometimes referred to simply as the "DNA of the present invention") encoding the protein of the present invention or its partial peptide, an antibody against the protein of the present invention, its partial peptide, or a salt thereof (hereinafter., sometimes referred to as the "antibody of the present invention") and an antisense polynucleotide to the polynucleotide (e.g., DNA) of the present invention (hereinafter, sometimes referred to simply as the "antisense polynucleotide of the present invention") will be specifically described for their applications.

The protein of the present invention has an acyltransferase activity. Hence, the protein of the present invention is useful as a marker for the early diagnosis in cancer tissues, judgment of severity in conditions, or predicted development of diseases. The compound or its salt that inhibits the activity of the protein of the present invention, the antibody against the protein of the present invention, the antisense polynucleotide of a gene encoding the protein of the present invention, or the siRNA, shRNA or the like of a gene encoding the protein of the present invention, can be used as, for example, a prophylactic/therapeutic agent for cancer (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

### (1) Screening of pharmaceutical agent candidate compounds for disease

The protein of the present invention has an acyltransferase activity of adding lipid to the N-terminus (e.g., Cys at the 24th position of the amino acid sequence represented by SEQ ID NO: 22) of Shh (e.g., human Shh, mouse Shh, etc.) which is increasingly expressed in cancer tissues. Therefore, a compound or its salt that inhibits an activity of the protein of the present invention is usable as a low toxicity cancer preventive/therapeutic agent, a cell apoptosis promoter, a cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

Accordingly, the protein of the present invention is useful as a reagent for screening the compound or its salt that inhibits the activity of the protein of the present invention.

That is, the present invention provides a method for screening a compound or its salt that inhibits the activity (e.g., the acyltransferase activity, etc.) of the protein of the present invention, which comprises using the protein of the present invention.

More specifically, there is employed a method for screening a compound or its salt that inhibits the activity of the protein of the present invention, which comprises comparing, e.g., (i) the acyltransferase activity of the protein of the present invention, with (ii) the acyltransferase activity of a mixture of the protein of the present invention and a test compound.

Specifically, (i') (a) the protein of the present invention, (b) a peptide at the N-terminus of Shh (e.g., human Shh, mouse Shh, etc.) (e.g., a peptide, or a partial peptide thereof, having an amino acid sequence comprised of the amino acids at the 24th position up to the 197th position of the amino acid sequence represented by SEQ ID NO: 22; etc.), and (c) palmitic acid which is radiolabeled (e.g., labeled with [¹²⁵I], [¹³¹I], [³H], [¹⁴C], [³²P], [³³P], [³⁵S], etc.) or fluorescence-labeled (e.g., labeled with a cyanine fluorescence dye (e.g., Cy2, Cy3, Cy5, Cy5.5, Cy7 (manufactured by Amersham Biosciences) etc.) are reacted. (ii') In the presence of a test compound, (a) the protein of the present invention, (b) a peptide at the N-terminus of Shh (e.g., human Shh, mouse Shh, etc.) (e.g., a peptide, or a partial peptide thereof, having an amino acid sequence including the amino acids at the 24th position up to the 197th position of the amino acid sequence represented by SEQ ID NO: 22; etc.), and (c) palmitic acid which is radiolabeled (e.g., labeled with [¹²⁵I], [¹³¹I], [³H], [¹⁴C], [³²P], [³³P], [³⁵S], etc.) or fluorescence-labeled (e.g., labeled with a cyanine fluorescence dye (e.g., Cy2, Cy3, Cy5, Cy5.5, Cy7 (manufactured by Amersham Biosciences) etc.) are reacted. In each of the cases of (i') and (ii'), the radioactivity or the fluorescent intensity derived from the palmitic acid added to the N-terminal peptide of Shh is measured. Thus, screening of a compound or its salt that inhibits the activity of the protein of the present invention is carried out. The reaction is carried out in an appropriate buffer. After the enzyme reaction, the N-terminal peptide of Shh is separated by, for example, SDS-polyacrylamide gel electrophoresis. Then, the radioactivity or the fluorescent intensity derived from the palmitic acid added to the peptide is measured. The radioactivity or the fluorescent intensity is measured by any known method using a scintillation counter, fluorography or the like. Instead of palmitic acid, myristic acid may be used.

The N-terminal peptide of Shh and the radiolabeled or fluorescence-labeled palmitic acid may be reacted with the protein of the present invention after being mixed with the test compound. Alternatively, after the N-terminal peptide of Shh and the radiolabeled or fluorescence-labeled palmitic acid are put into contact with the protein of the present invention, the test compound may be added. Myristic acid may be used in substantially the same manner as the palmitic acid.

As compared with the protein or peptide which is modified with lipid, a protein or peptide which is not modified with lipid through acyltransferase remains in the cell at a higher possibility. This property can be used to perform screening of a compound inhibiting the activity of the protein of the present invention.

Specifically, in the presence of a peptide at the N-terminus of Shh (e.g., human Shh, mouse Shh, etc.) (e.g., a peptide, or a partial peptide thereof, having an amino acid sequence including the amino acids at the 24th position up to the 197th position of the amino acid sequence represented by SEQ ID NO: 22; etc.), the Shh being fluorescence-labeled (e.g., a cyanine fluorescence dye (e.g., Cy2, Cy3, Cy5, Cy5.5, Cy7 (manufactured by Amersham Biosciences) etc.), (i") cells having an ability of producing the protein of the present invention are cultured, and (ii") cells having an ability of producing the protein of the present invention are cultured in the presence of the test compound. In each of the cases of (i") and (ii"), the fluorescent intensity of the cell and the cell density are measured to calculate the fluorescent intensity ratio per cell. Thus, screening of a compound or its salt that inhibits an activity of the protein of the present invention is performed.

The fluorescent intensity is measured in accordance with any known method using fluorography or the like. The cell density is measured in accordance with any known method using a nuclear dye reagent (e.g., DRAQ5 (Biostatus)) or the like.

Preferably, the protein of the present invention described above is produced by culturing transformants containing DNA encoding the protein of the present invention. Furthermore, the reaction is similarly performed using cells capable of expressing the protein of the present invention.

As the cells capable of producing the protein of the present invention, there are used, for example, a host (transformant) transformed with a vector containing the DNA encoding the protein of the present invention. Preferably, animal cells such as COS7 cells, CHO cells, HEK293 cells, etc. are used as the host. For the screening, the transformant, in which the protein of the present invention has been expressed by culturing through the procedures described above, is preferably employed. The procedures for culturing the cells capable of expressing the protein of the present invention are similar to the culturing procedures for the transformant of the present invention described above.

Examples of the test compound include peptides, proteins, antibodies, non-peptide compounds, synthetic compounds, fermentation products, cell extracts, plant extracts, animal tissue extracts, plasma, etc.

For example, a test compound which decreases the acyltransferase activity in the case of (ii) (including (ii") and (ii")) described above by at least about 20%, preferably at least 30% and more preferably at least about 50%, as compared to the case of (i) (including (i") and (i")) described above, can be selected as the compound capable of inhibiting the activity of the protein of the present invention.

The compound having the activity of inhibiting the activity of the protein of the present invention is useful as a safe and low toxicity pharmaceutical agent to suppress the physiological activity of the protein of the present invention, such as a cancer preventive/therapeutic agent, a cell apoptosis promoter, a cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

The compound or its salt obtained using the screening method or screening kit of the present invention is a compound selected from, for example, peptides, proteins, antibodies, non-peptide compounds, synthetic compounds, fermentation products, cell extracts, plant extracts, animal tissue extracts, plasma, etc. The usable salts of these compounds are substantially the same as those given above as the salts of the peptide of the present invention.

In addition, the gene encoding the protein of the present invention also produces a protein having an acyltransferase activity. Therefore, a compound or its salt that inhibits the expression of the gene encoding the protein of the present invention can be used as a cancer preventive/therapeutic agent, a cancer cell apoptosis promoter, a cancer cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

Therefore, the DNA of the present invention is useful as a reagent for screening a compound or its salt that inhibits the expression of the gene encoding the protein of the present invention.

For the screening, there is a method of screening which comprises comparing (iii) the case where a cell capable of producing the protein of the present invention is cultured and (iv) the case where a cell capable of producing the protein used in the present invention is cultured in the presence of a test compound.

In the method described above, the expression level of the gene described above (specifically, the level of the protein of the present invention or the level of mRNA encoding the above protein) is determined in the cases of (iii) and (iv), followed by comparison.

Examples of the test compound and the cells capable of producing the protein of the present invention are substantially the same as described above.

The level of the protein of the present invention can be determined by known methods, e.g., by measuring the aforesaid protein present in the cell extract, etc., using an antibody capable of recognizing the protein, in accordance with a method such as Western blot analysis, ELISA, etc., or a method confirmed thereto.

The mRNA level can be determined by any known method, for example, Northern hybridization using a nucleic acid containing the entirety or a part of the amino acid sequence represented by SEQ ID NO: 2, SEQ ID NO: 4 or SEQ ID NO: 6 as a probe, PCR using a nucleic acid containing the entirety or a part of the amino acid sequence represented by SEQ ID NO: 2, SEQ ID NO: 4 or SEQ ID NO: 6 as a primer, or a method conformed thereto.

For example, a test compound which inhibits the expression level of the gene in the case (iv) described above by at least about 20%, preferably at least 30% and more preferably at least about 50%, as compared to the case (iii) above can be selected to be a compound capable of suppressing (inhibiting) the expression of the gene encoding the protein of the present invention.

The screening kit of the present invention comprises the protein used in the present invention, its partial peptide or salts thereof, or a cell capable of producing the protein used in the present invention, or its partial peptide.

The compound or its salt obtained by using the screening method or screening kit of the present invention is the test compound described above, e.g., a compound selected from peptides, proteins, antibodies, non-peptide compounds, synthetic compounds, fermentation products, cell extracts, plant extracts, animal tissue extracts, plasma, etc., or its salt, which is a compound or its salt inhibiting the activity of the protein of the present invention (e.g., the acyltransferase activity, etc.), the expression of the gene for the protein of the present invention, or the expression of the protein of the present invention.

The usable salts of these compounds are substantially the same as those given above as the salts of the protein of the present invention.

A compound or a salt thereof that inhibits the activity of the protein of the present invention, a compound or a salt thereof that inhibits the expression of a gene encoding the protein of the present invention, or a compound or a salt thereof that inhibits the expression of the protein of the present invention, are low in toxicity and useful as a cancer preventive/therapeutic agent, a cancer cell apoptosis promoter, a cancer cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

Where the compound or its salt obtained by using the screening method or screening kit of the present invention is used as a the prophylactic/therapeutic agent described above, such a compound can be converted into a pharmaceutical preparation in a conventional manner.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by any known method and contains a carrier, a diluent or an excipient conventionally used in the field of pharmaceutical preparations. Examples of the carrier or excipient for tablets include lactose, starch, sucrose, magnesium stearate, etc.

Examples of the composition for parenteral administration include injectable preparations, suppositories, etc. Examples of the injectable preparations may include dosage forms such as intravenous, subcutaneous, intracutaneous and intramuscular injections, drip infusions, intraarticular injection, etc. These injectable preparations may be prepared by any known method. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually put into an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the antibody contained is generally 5 to 500 mg per dosage unit form; and is especially about 5 to about 100 mg in the form of injection, and 10 to 250 mg in the other forms.

Each composition described above may further contain other active components unless the formulation causes any adverse interaction with the compound described above.

Since the pharmaceutical preparations thus obtained are safe and low in toxicity, they can be administered to human or warm-blooded animal (e.g., mouse, rat, rabbit, ovine, swine, bovine, equine, fowl, feline, canine, simian, chimpanzee, etc.) orally or parenterally.

The dose of the compound or its salt may vary depending upon its action, target disease, subject to be administered, administration route, etc. For example, when a compound or its salt that inhibits the activity of the protein of the present invention is orally administered for the purpose of treating, e.g., colorectal cancer, the compound or its salt is generally administered to an adult (body weight: 60 kg) in a daily dose of about 0.1 to about 100 mg, preferably about 1.0 to about 50 mg and more preferably about 1.0 to about 20 mg. In the case of parenteral administration, a single dose of the compound or its salt may vary depending upon subject to be administered, target disease, etc. When a compound or its salt that inhibits the activity of the protein of the present invention is administered to an adult (body weight: 60 kg) in the form of an injectable preparation for the purpose of treating, e.g., breast cancer, it is advantageous to administer the compound or its salt to the affected area in a daily dose of about 0.01 to about 30 mg, preferably about 0.1 to about 20 mg, and more preferably about 0.1 to about 10 mg. For other animal species, the corresponding dose as converted based on the body weight of 60 kg can be administered.

Also, the compounds described above can be used in combination with a known antitumor agent, for example, an alkylating agent (e.g., cyclophosphamide, busulfan, merphalan, etc.), a metabolic antagonist (e.g., cytarabine, 6-mercaptopurine, methotrexate, 5- fluorouracil, etc.), an antitumor antibiotic (e.g., daunorubicin, doxorubicin, pirarubicin, mitoxantron, idarubicin, mitomycin, adriamycin, etc.), a plant derived antitumor agent (e.g., vincristine, vindesine, taxol, etoposide, etc.), an enzyme preparation (e.g., L-asparaginase, etc.), an adrenocortical hormone agent (e.g., prednisolone, prednisone, dexamethasone, cortisone acetate, etc.), an estrogen drug (e.g., estradiol, ethinyl estradiol, fosfestrol, chlorotrianisene, etc.), an anti-estrogen drug (e.g., epithiostanol, mepitiostane, tamoxifen, clomifene, etc.), a progesterone drug (e.g., hydroxyprogesterone caproate, dydrogesterone, medroxyprogesterone, norethisterone, norethindrone, etc.), an LHRH derivative (e.g., leuprorelin acetate, etc.), cisplatin, carboplatin, transretinoic acid, interferon α, imatinib, or the like. The timing of administration is not limited; these agents may be administered to the target subject simultaneously or at staggered times. The dose may be appropriately chosen on the dose which is clinically applied. A ratio of the compound described above to the antitumor agent can be appropriately selected, depending on the subject to be administered, administration route, target disease, clinical conditions, combination, etc.

### (2) Quantification for the protein of the present invention, its partial peptide or its salt

The antibody against the protein of the present invention (hereinafter, sometimes referred to simply as the "antibody of the present invention") is capable of specifically recognizing the protein of the present invention, and thus can be used for quantification of the protein of the present invention in a test sample fluid, in particular, for quantification by sandwich immunoassay, etc.

That is, the present invention provides:
(i) A method for quantifying the protein of the present invention in a test sample fluid, which comprises competitively reacting the antibody of the present invention, a test sample fluid and a labeled form of the protein of the present invention; and measuring the ratio of the labeled form of the protein of the present invention bound to said antibody; and,
(ii) A method for quantifying the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody against the protein of the present invention (hereinafter, sometimes referred to as the "monoclonal antibody of the present invention") can be used to quantify the protein of the present invention. In addition, the protein can be detected by means of a tissue staining or the like. For these purposes, the antibody molecule per se may be used or F(ab')₂, Fab' or Fab fractions of the antibody molecule may also be used.

The method for quantifying the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described below.

Examples of the labeling agent used in the assay method using the labeling substance include radioisotopes (e.g., [¹²⁵I], [¹³¹I], [³H], [¹⁴C], [³²P], [³³P], [³⁵S], etc.), fluorescent substances (e.g., cyanine fluorescence dyes (e.g., Cy2, Cy3, Cy5, Cy5.5, Cy7 (manufactured by Amersham Biosciences, Inc.) etc.), fluorescamine, fluorescein isothiocyanate, NBD (7-nitrobenz-2-oxa-1,3-diazol), etc.), enzymes (e.g., β-galactosidase, β-glucosidase, alkaline phosphatase, peroxidase, malate dehydrogenase, etc.), luminescent substances (e.g., luminol, luminol derivatives, luciferin, lucigenin, etc.), biotin, lanthanide element, etc. Furthermore, the biotin-avidin system may be used as well for binding of an antibody or antigen to a labeling agent.

For insolubilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resins such as polystyrene, polyacrylamide, silicone, etc., and glass or the like.

By the sandwich method, the insolubilized monoclonal antibody of the present invention is reacted with a test fluid (primary reaction), then with a labeled form of another monoclonal antibody of the present invention (secondary reaction), and the activity of the label on the insolubilizing carrier is measured. Thus, the amount of the protein of the present invention in the test fluid can be quantified. The order of the primary and secondary reactions may be reversed, and the reactions may be performed simultaneously or with an interval. The labeling and insolubilization can be performed by a method conformed to any of the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method of the present invention, antibodies that bind to different sites of the protein of the present invention are preferably used as the monoclonal antibodies of the present invention used for the primary and secondary reactions. That is, for example, when the antibody used in the secondary reaction recognizes the C-terminal region of the protein of the present invention, the antibody used for the primary reaction is the one recognizing the region other than the C-terminal region, e.g., the antibody recognizing the N-terminal region.

The monoclonal antibodies of the present invention can be used for the assay systems other than the sandwich method, for example, the competitive method, the immunometric method, nephrometry, etc.

In the competitive method, the antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. Usable for this reaction method are, for example, a liquid phase method using a soluble antibody as an antibody, polyethylene glycol for B/F separation and a secondary antibody against the soluble antibody, and an immobilized method either using an immobilized antibody as the primary antibody, or using a soluble antibody as the primary antibody and immobilized antibody as the secondary antibody.

In the immunometric method, the antigen in a test fluid and the immobilized antigen are competitively reacted with a definite amount of labeled antibody, the immobilized phase is separated from the liquid phase, or the antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced as a result of the antigen-antibody reaction in gel or solution is quantified. Even when the amount of antigen in the test fluid is small and only a small amount of precipitate is obtained, laser nephrometry using scattering of laser is advantageously employed.

For applying each of these immunological methods to the quantification method of the present invention, any particular conditions or procedures are not required. A quantification system for the protein of the present invention may be constructed by adding the usual technical consideration in the art to the conventional conditions and procedures. For the details of these general technical means, reference can be made to the following reviews and textbooks.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the protein of the present invention can be quantified with high sensitivity, using the antibody of the present invention.

Furthermore, when an increased or decreased concentration of the protein of the present invention is detected by quantifying the concentration of the protein of the present invention using the antibody of the present invention, it can be diagnosed that the patient suffers from, or the patient will highly likely suffer in the future from, for example, cancer (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; typically, medulloblastoma, breast cancer, small cell lung cancer, gastric cancer, esophageal cancer, colorectal cancer, colon cancer, pancreatic cancer, bile duct cancer, prostate cancer, etc.).

In addition, the antibody of the present invention can be used to detect the protein of the present invention, which is present in a test sample such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

### (3) Gene diagnostic agent

The DNA of the present invention, when used as, for example, a probe, can detect an abnormality (gene abnormality) of the DNA or mRNA encoding the protein of the present invention or its partial peptide in human or warm-blooded animal (e.g., rat, mouse, guinea pig, rabbit, fowl, ovine, swine, bovine, equine, feline, canine, simian, chimpanzee, etc.). Therefore, the DNA of the present invention is useful as a gene diagnostic agent for detecting damages to the DNA or mRNA, or mutation, decreased expression, increased expression, overexpression, etc. of the DNA or mRNA, or the like.

The gene diagnosis described above using the DNA of the present invention can be performed by, for example, the known Northern hybridization assay or the PCR-SSCP assay (Genomics, 5, 874-879 (1989); Proceedings of the National Academy of Sciences of the United States of America, 86, 2766-2770 (1989)), etc.

When overexpression is detected by, e.g., Northern hybridization or DNA mutation is detected by the PCR-SSCP assay, it can be diagnosed that the patient highly likely suffers from, for example, cancer (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; typically, medulloblastoma, breast cancer, small cell lung cancer, gastric cancer, esophageal cancer, colorectal cancer, colon cancer, pancreatic cancer, bile duct cancer, prostate cancer, etc.).

### (4) Pharmaceutical agent comprising the antisense polynucleotide

The antisense polynucleotide of the present invention that binds to the DNA of the present invention complementarily to suppress the expression of said DNA has the effect of suppressing the proliferation of cancer cells, promoting/increasing apoptosis of cancer cells, or the like, is low in toxicity, and can suppress the function (e.g., acyltransferase activity) of the protein of the present invention or the DNA of the present invention in vivo. Therefore, the antisense polynucleotide of the present invention can be used, for example, as a preventive/therapeutic agent, a cell apoptosis promoter, a cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

Where the antisense polynucleotide described above is used as the aforesaid prophylactic/therapeutic agent, it can be prepared into pharmaceutical preparations by known methods, which are provided for administration.

For example, the antisense polynucleotide described above may be administered orally or parenterally to human or mammal (e.g., rat, rabbit, ovine, swine, bovine, feline, canine, simian, etc.) in a conventional manner after being inserted into an appropriate vector such as retrovirus vector, adenovirus vector, adenovirus-associated virus vector, etc. The antisense polynucleotide may also be administered as it stands, or may be prepared in pharmaceutical preparations together with a physiologically acceptable carrier for assisting its uptake, which are then administered by a gene gun or through a catheter with a hydrogel or other types of catheters. Alternatively, the antisense polynucleotide may be prepared into an aerosol, which is topically administered into the trachea as an inhaler.

Further for the purposes of improving pharmacokinetics, extending a half-life and improving the intracellular uptake efficiency, the antisense polynucleotide described above may be prepared into pharmaceutical preparations (injectable preparations) alone or together with a carrier such as liposome, etc. and the preparations may be administered intravenously, subcutaneously, in articular cavities or at the affected area, etc.

A dose of the antisense polynucleotide may vary depending on target disease, subject to be administered, administration route, etc. For example, where the antisense polynucleotide of the present invention is administered for the purpose of treating colorectal cancer, the antisense polynucleotide is generally administered to an adult (body weight: 60 kg) in a daily dose of about 0.1 to 100 mg.

Furthermore, the antisense polynucleotide may also be used as an oligonucleotide probe for diagnosis to examine the presence of the DNA of the present invention in tissues or cells and the states of its expression.

As in the antisense polynucleotide described above, the double-stranded RNA containing a part of RNA encoding the protein of the present invention (siRNA (small (short) interfering RNA to the polynucleotide of the present invention), shRNA (small (short) hairpin RNA, etc.), ribozyme containing a part of RNA encoding the protein of the present invention, etc. can also prevent the expression of the gene encoding the present invention to suppress the in vivo function of the protein used in the present invention or the DNA used in the present invention and hence can be used, for example, as a preventive/therapeutic agent, a cell apoptosis promoter, a cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

The double-stranded RNA can be designed based on a sequence of the polynucleotide of the present invention and manufactured by a known method (e.g., Nature, 411, 494, 2001) or a method conformed thereto.

The ribozyme can be designed based on a sequence of the polynucleotide of the present invention and manufactured by a known method (e.g., TRENDS in Molecular Medicine, 7, 221, 2001) or a method conformed thereto. For example, the ribozyme can be manufactured by ligating a known ribozyme to a part of the RNA encoding the protein of the present invention. A part of the RNA encoding the protein of the present invention may be, for example, a portion proximal to a cleavage site on the RNA of the present invention, which may be cleaved by a known ribozyme (RNA fragment).

For using the double-stranded RNA or ribozyme described above as the prophylactic/therapeutic agent described above, the double-stranded RNA or ribozyme may be prepared into pharmaceutical preparations and administered in substantially the same manner as the antisense polynucleotide.

### (5) Pharmaceutical agent comprising the antibody of the present invention

The antibody of the present invention, which has the activity of neutralizing the activity of the protein of the present invention can be used, for example, as a preventive/therapeutic agent, a cell apoptosis promoter, a cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

The prophylactic/therapeutic agent comprising the antibody of the present invention for the diseases described above is low in toxicity and can be administered to human or mammals (e.g., rat, rabbit, ovine, swine, bovine, feline, canine, simian, etc.) orally or parenterally (e.g., intravenously) in the form of a liquid preparation as it is or as a pharmaceutical composition of appropriate dosage form. The dose may vary depending upon the subject to be administered, target disease, conditions, administration route, etc. For example, when the agent is used for the purpose of treating, e.g., colorectal cancer in an adult, it is advantageous to administer the antibody of the present invention normally in a single dose of about 0.01 to about 20 mg/kg body weight, preferably about 0.1 to about 10 mg/kg body weight, and more preferably about 0.1 to about 5 mg/kg body weight, approximately 1 to 5 times per day, preferably approximately 1 to 3 times per day. In other parenteral administrations and oral administration, the agent can be administered in a dose corresponding to the dose given above. When the condition is especially severe, the dose may be increased according to the condition.

The antibody of the present invention can be administered directly or as a pharmaceutical composition of appropriate dosage form. A pharmaceutical composition used for the administration comprises the antibody described above or its salt, a pharmaceutically acceptable carrier, dilute or excipient. Such a composition is provided as a dosage form appropriate for oral or parenteral (e.g., intravenous) administration, preferably, as an inhaler.

Each composition described above may further contain other active components, unless their formulation with the antibody causes any adverse interaction.

### (6) DNA transgenic animal

The present invention provides a non-human mammal bearing DNA encoding a protein of the present invention, which is exogenous (hereinafter, referred to simply as the "exogenous DNA of the present invention") or its variant DNA (sometimes referred to simply as the "exogenous variant DNA of the present invention").

That is, the present invention provides:
(1) A non-human mammal bearing an exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing an exogenous DNA of the present invention or its variant DNA and capable of expressing in a mammal.

The non-human mammal bearing the exogenous DNA of the present invention or its variant DNA (hereinafter, referred to simply as the "DNA transgenic animal of the present invention") can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and to utilize the transformant for cell culture, tissue culture, etc. In addition, such a cell may be fused with the above-described germinal cell by a known cell fusion method to prepare the DNA transgenic animal of the present invention.

Examples of the non-human mammal that can be used here include bovine, swine, ovine, goat, rabbit, canine, feline, guinea pig, hamster, mouse, rat, etc. Preferred among them are rodents, especially mouse (e.g., for a pure line, C57BL/6 strain, DBA2 strain, etc.; and for a cross line, B6C3F₁ strain, BDF₁ strain, B6D2F₁ strain, BALB/c strain, ICR strain, etc.), rat (Wistar, SD, etc.) and the like. They have relatively short ontogeny and life cycles and easy to breed and therefore are preferred from a viewpoint of creating model animals for human disease.

"Mammals" regarding a recombinant vector that can be expressed in the mammals include the aforesaid non-human mammals, human, etc.

The exogenous DNA of the present invention refers to the DNA of the present invention that is once isolated/extracted from mammals, not the DNA of the present invention inherently possessed by the non-human mammals.

The "variant DNA" of the present invention encompasses DNA resulting from variation (e.g., mutation, etc.) in the nucleotide sequence of the original DNA of the present invention, specifically DNAs resulting from nucleotide addition, deletion, substitution with other nucleotides, etc. and further includes abnormal DNA.

The "abnormal DNA" is intended to mean DNA that expresses a protein of the present invention which is abnormal, for example, DNA that expresses a protein for suppressing the function of a normal protein of the present invention.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct ligated downstream a promoter capable of expressing the DNA in the target animal. For example, in the case of transfecting the human DNA of the present invention, a DNA transgenic mammal that expresses the DNA of the present invention to a high level can be prepared by microinjecting a DNA construct (e.g., vector, etc.) ligated with the human DNA of the present invention into a fertilized egg (e.g., mouse fertilized egg) of the target non-human mammal downstream any of various promoters which are capable of expressing the DNA derived from any of various mammals (e.g., rabbit, canine, feline, guinea pig, hamster, rat, mouse, etc.) bearing the DNA of the present invention highly homologous to the human DNA.

As expression vectors usable for the protein of the present invention, there are Escherichia coli-derived plasmids, Bacillus subtilis-derived plasmids, yeast-derived plasmids, bacteriophages such as λ phage, etc., retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbit, canine, feline, guinea pig, hamster, rat, mouse, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as "Tie2"), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a "terminator"); for example, a sequence of each DNA derived from viruses and various mammals, preferably SV40 terminator of the simian virus, etc.

In addition, for the purpose of enhancing the expression of the desired exogenous DNA to a higher level, the splicing signal and enhancer region of each DNA, a portion of the intron of an eukaryotic DNA, etc. may also be ligated at the 5' upstream of the promoter region, or between the promoter region and the translational region, or at the 3' downstream of the translational region, depending upon purposes.

The translational region for the normal protein of the present invention can be obtained using, as a starting material, the entire genomic DNA or its portion of liver, kidney, thyroid cell or fibroblast origin from human or various mammals (e.g., rabbit, canine, feline, guinea pig, hamster, rat, mouse, etc.) or of various commercially available genomic DNA libraries, or using cDNA prepared by a known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all the offspring of the prepared animal will maintain the exogenous DNA of the present invention in all of the germinal cells and somatic cells thereof. The offspring of the animal that inherits the exogenous DNA of the present invention also has the exogenous DNA of the present invention in all of the germinal cells and somatic cells thereof.

The non-human mammal in which the normal exogenous DNA of the present invention has been transfected can be passaged as the DNA-bearing animal under normal breeding environment, after it is confirmed that the exogenous DNA is stably retained by crossing.

By the transfection of the exogenous DNA of the present invention at the fertilized egg cell stage, the DNA is excessively retained in all of the germinal and somatic cells in the target mammal. The fact that the exogenous DNA of the present invention is excessively present in the germinal cells of the prepared animal after transfection means that all the offspring of the prepared animal will have the exogenous DNA of the present invention excessively in all of the germinal cells and somatic cells thereof. The offspring of the animal that inherits the exogenous DNA of the present invention has the exogenous DNA of the present invention excessively in all of the germinal cells and somatic cells thereof.

It is possible to obtain homozygotic animals having the transfected DNA in both homologous chromosomes and breed male and female of the animal so that all the progeny has this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, a mammal transfected with the exogenous normal DNA of the present invention exhibits a symptom of increasing the protein of the present invention, which is liberated. Thus, the animal is available for screening test of, for example, a preventive/therapeutic agent, a cell apoptosis promoter, a cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

On the other hand, a non-human mammal having the exogenous abnormal DNA of the present invention can be passaged under normal breeding environment as the DNA-bearing animal, after it is confirmed that the exogenous DNA is stably retained by crossing. Furthermore, the desired exogenous DNA can be utilized as a starting material by being inserted into the plasmid described above. The DNA construct with a promoter can be prepared by a conventional DNA engineering technique. The abnormal DNA of the present invention is transfected at the fertilized egg cell stage in a manner such that the DNA is certainly present in all the germinal cells and somatic cells of the target mammal. The fact that the abnormal DNA of the present invention is present in the germinal cells of the animal prepared by DNA transfection means that all the offspring of the prepared animal will maintain the abnormal DNA of the present invention in all of the germinal cells and somatic cells thereof. The offspring of the animal that inherits the abnormal DNA of the present invention has the abnormal DNA of the present invention in all of the germinal cells and somatic cells thereof. It is possible to obtain homozygotic animals having the transfected DNA in both homologous chromosomes and breed male and female of the animal so that all the progeny has this DNA in excess.

In a non-human mammal bearing the abnormal DNA of the present invention, the abnormal DNA of the present invention is overexpressed, and may eventually develop the function inactive type inadaptability to the protein of the present invention by inhibiting the function of endogenous normal DNA. Therefore, the animal can be utilized as a pathologic model animal for such a disease. For example, using the abnormal DNA transgenic animal of the present invention, it is possible to elucidate the pathological mechanism of the function inactive type inadaptability to the protein of the present invention and investigate how to treat this disease.

As a specific example of the applicability, the transgenic animal overexpressing the abnormal DNA of the present invention is expected to serve as an experimental model to elucidate the mechanism of the functional inhibition (dominant negative effect) of a normal protein caused by the abnormal protein of the present invention in the function inactive type inadaptability to the protein of the present invention.

A mammal bearing the abnormal exogenous DNA of the present invention is also available for the screening test of prophylactic/therapeutic agents for the function inactive type inadaptability to the protein of the present invention, e.g., a preventive/therapeutic agent, a cell apoptosis promoter, a cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

Other potential applications of the two kinds of the DNA transgenic animals of the present invention described above further include:
(i) Use as a cell source for tissue culture;
(ii) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
(iii) Research on the function of cells derived from tissues that are usually difficult to be cultured, using cells in tissues bearing the DNA cultured by a standard tissue culture technique;
(iv) Screening of an agent that enhances the function of cells using the cells described in (iii) above; and,
(v) Isolation and purification of the variant protein of the present invention and preparation of an antibody thereto; etc.

Furthermore, clinical conditions of a disease associated with the protein of the present invention, including the function inactive type inadaptability to the protein of the present invention, can be determined by using the DNA transgenic animal of the present invention. Also, pathological findings on each organ in a disease model associated with the protein of the present invention can be obtained in more detail, which may contribute to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading the organ with a proteinase such as trypsin, etc., and thus to establish the line of culturing or cultured cells. Furthermore, the DNA transgenic animal can serve to identify cells capable of producing the protein of the present invention, and to study the association with apoptosis, differentiation or propagation or the mechanism of signal transduction in these properties to inspect any abnormality therein. Accordingly, the DNA transgenic animal can provide an effective research material for the protein of the present invention and for investigation of the function and effect thereof.

To develop a therapeutic agent for the treatment of diseases associated with the protein of the present invention, including the function inactive type inadaptability to the protein of the present invention, using the DNA transgenic animal of the present invention, an effective and rapid method for screening can be provided by using any of the inspection and quantification methods, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (7) Knockout animal

The present invention provides a non-human mammal embryonic stem cell in which the DNA of the present invention is inactivated and a non-human mammal deficient in expression of the DNA of the present invention.

Thus, the present invention provides:
(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from Escherichia coli);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expression of the DNA of the present invention, wherein the DNA is inactivated;
(7) The non-human mammal according to (6), wherein the DNA is inactivated by introducing a reporter gene (e.g., β-galactosidase derived from Escherichia coli) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound or its salt that promotes or inhibits a promoter activity to the DNA of the present invention, which comprises administering a test compound to the mammal of (7) and detecting expression of the reporter gene.

The "non-human mammal embryonic stem cell in which the DNA of the present invention is inactivated" refers to a non-human mammal embryonic stem cell in which the ability of the non-human mammal to express the DNA is suppressed by artificially mutating the DNA of the present invention, or a non-human mammal embryonic stem cell in which the DNA has no substantial ability to express the protein of the present invention (hereinafter, sometimes referred to as the "knockout DNA of the present invention") by substantially inactivating the activities of the protein of the present invention encoded by the DNA (hereinafter, the "non-human mammal embryonic stem cell in which the DNA of the present invention is inactivated" will be referred to simply as the "ES cell").

As the non-human mammal, the same mammals as those described above are usable.

Techniques for artificially mutating the DNA of the present invention include deletion of a part or the entirety of the DNA sequence, insertion of or substitution with other DNA, etc., which is performed by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter, referred to simply as the "DNA-inactivated ES cell of the present invention" or the "knockout ES cell of the present invention") can be obtained, for example, as follows. The DNA of the present invention that the desired non-human mammal possesses is isolated. Then, a DNA chain having a DNA sequence constructed as follows is introduced to a chromosome of the target animal by, e.g., homologous recombination (hereinafter, such a DNA chain will be referred to as the "targeting vector"). The DNA sequence is constructed by (a) inserting a drug resistant gene such as a neomycin resistant gene or a hygromycin resistant gene, or a reporter gene such as lacZ (β-galactosidase gene) or cat (chloramphenicol acetyltransferase gene), etc. into the exon site of the isolated DNA, thereby disabling the functions of exon, or (b) inserting a DNA sequence that terminates gene transcription (e.g., polyA additional signal, etc.) into the intron between exons, thus inhibiting the synthesis of complete messenger RNA so as to destroy the gene. The ES cells thus obtained is analyzed by the Southern hybridization analysis using a DNA sequence on or near the DNA of the present invention as a probe, or by the PCR analysis using a DNA sequence on the targeting vector and another DNA sequence near the DNA of the present invention which is not included in the targeting vector as primers. Thus, the knockout ES cell of the present invention is selected.

The parent ES cells for inactivating the DNA of the present invention by homologous recombination, etc. may be of a strain already established as described above, or may be newly established in accordance with any known method such as the Evans and Kaufman method or a method conformed thereto. For example, in the case of mouse ES cells, it is currently common practice to use ES cells of the 129 strain. However, since their immunological background is obscure, the C57BL/6 mouse or the BDF₁ mouse (F₁ between C57BL/6 and DBA/2) obtained by improving the low ovum availability per animal of the C57BL/6 mouse through crossing with DBA/2, may be preferably used, for the purpose of, for example, obtaining a pure line of ES cells with the clear immunological genetic background replacing the ES cells of the 129 strain. The BDF₁ mouse is advantageous in that ovum availability per animal is high and ova are robust, and also for the following point. BDF₁ mouse has the C57BL/6 mouse as a background. Therefore, when a pathologic model mouse is generated using ES cells obtained therefrom, the genetic background can be changed to that of the C57BL/6 mouse by back-crossing with the C57BL/6 mouse.

In establishing ES cells, blastocytes at 3.5 days after fertilization are commonly used. In addition thereto, embryos are preferably collected at the 8-cell stage, cultured until the blastocyte stage and then used. In this way, a large number of early stage embryos can be obtained efficiently.

Although the ES cells used may be of either sex, male ES cells are generally more convenient for generation of a germ cell line chimera. It is also desirable that sexes are identified as soon as possible to save painstaking culture time.

Methods for sex identification of the ES cell include a method in which a gene in the sex-determining region on the Y-chromosome is amplified by the PCR process and detected. When this method is used, about one colony of ES cells (about 50 cells) is sufficient for sex-determination analysis, instead of about 10⁶ cells conventionally required by karyotype analysis. Therefore, the first selection of ES cells at the early stage of culture can be based on sex identification, and male cells can be selected on an early stage, which saves a significant amount of time at the early stage of culture.

Also, second selection can be achieved by, for example, confirmation of the number of chromosomes by the G-banding method. It is usually desirable that the number chromosomes of the obtained ES cells be 100% of the normal number. However, when it is difficult to obtain the cells having the normal number of chromosomes due to physical operations, etc. in the cell establishment, it is desirable that the ES cell be again cloned to a normal cell (e.g., in a mouse, a cell having the number of chromosomes of 2n = 40) after knockout of the gene of the ES cells.

Although the embryonic stem cell line thus obtained usually shows a very high growth potential, it must be subcultured with great care, since it tends to easily lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, and treated with, for example, a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, parietal, visceral, or cardiac muscle [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology and Experimental Morphology, 87, 27, 1985]. The cells deficient in expression of the DNA of the present invention, which are obtained from the differentiated ES cells of the present invention, are useful for cytological study of the protein of the present invention in vitro.

The non-human mammal deficient in expression of the DNA of the present invention can be identified from a normal animal by measuring the mRNA level in the subject animal by a known method, and indirectly comparing the degrees of expression.

As the non-human mammal, substantially the same mammals as those given above are usable.

With respect to the non-human mammal deficient in expression of the DNA of the present invention, the DNA of the present invention can be knocked out by transfecting a targeting vector prepared as described above to mouse embryonic stem cells or mouse oocytes, and conducting homologous recombination, more specifically by replacing a targeting vector DNA sequence, wherein the DNA of the present invention is inactivated by the transfection, with the DNA of the present invention on a chromosome of a mouse embryonic stem cell or mouse embryo.

The knockout cells with the disrupted DNA of the present invention can be identified by the Southern hybridization analysis using a DNA sequence on or near the DNA of the present invention as a probe, or by the PCR analysis using a DNA sequence on the targeting vector and another DNA sequence near the DNA of the present invention which is not included in the targeting vector as primers. When non-human mammal stem cells are used, a cell line in which the DNA of the present invention is inactivated by homologous recombination is cloned and the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both of cells having the normal locus of the DNA of the present invention and cells having an artificially mutated locus of the DNA of the present invention.

When some germ cells of the chimeric animal have a mutated locus of the DNA of the present invention, individuals, in which the entire tissue is composed of cells having an artificially mutated locus of the DNA of the present invention can be selected from a group of individuals obtained by crossing between such a chimeric animal and a normal animal by, for example, coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the oocyte nucleus by microinjection to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knocked out can be passaged under normal breeding environment, after it is confirmed that the individuals obtained by their crossing also has the knockout DNA.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell in which the DNA of the present invention is inactivated is very useful for preparing a non-human mammal deficient in expression of the DNA of the present invention.

Since the non-human mammal in which the DNA of the present invention is inactivated lacks various biological activities derived from the protein of the present invention, such an animal can be a disease model suspected to be caused by inactivated biological activities of the protein of the present invention and thus, offers an effective study to investigate the causes for and therapy for these diseases.

### (7a) Method for screening a compound having a therapeutic/prophylactic effect on diseases caused by deficiencies, damages, etc. of the DNA of the present invention

The non-human mammal deficient in expression of the DNA of the present invention can be employed for screening a compound having a therapeutic/prophylactic effect on diseases caused by deficiencies, damages, etc. of the DNA of the present invention.

That is, the present invention provides a method for screening a compound or its salt having an effect of treating/preventing a disease caused by deficiencies, damages, etc. of the DNA of the present invention, e.g., cancer, etc., which comprises administering a test compound to a non-human mammal deficient in expression of the DNA of the present invention, and observing/determining changes in the animal.

The non-human mammal deficient in expression of the DNA of the present invention, which can be employed for the screening method, may be substantially the same mammals as those described above.

Examples of the test compound include peptides, proteins, antibodies, non-peptide compounds, synthetic compounds, fermentation products, cell extracts, plant extracts, animal tissue extracts, blood plasma, etc. These compounds may be novel compounds or known compounds.

Specifically, the non-human mammal deficient in expression of the DNA of the present invention is treated with a test compound, compared with an intact animal for control. A change in each of organs, tissues, disease conditions, etc. of the control animal is used as an indicator to assess the therapeutic/prophylactic effects of the test compound.

For treating a test animal with a test compound, for example, oral administration, intravenous injection, etc. is applicable and appropriately selected depending on conditions of the test animal, properties of the test compound, etc. Furthermore, a dose of the test compound to be administered can be appropriately chosen depending on the administration method, property of the test compound, etc.

For screening a compound having an effect of treating/preventing, for example, cancer (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.), a test compound is administered to the non-human mammal deficient in expression of the DNA of the present invention. Differences in incidence of cancer or differences in degree of healing from the group administered with no test compound are observed in the tissues described above with passage of time.

In the screening method, when a test compound is administered to a test animal and the disease conditions of the test animal are improved by at least about 10%, preferably at least about 30% and more preferably at least about 50%, the test compound can be selected as a compound having a therapeutic/prophylactic effect on the diseases described above.

The compound obtained using the above screening method is a compound selected from the test compounds described above and exhibits therapeutic/prophylactic effect on diseases caused by deficiencies, damages, etc. of the protein of the present invention. Therefore, the compound can be employed as a safe and low toxicity pharmaceutical agent, for example, a prophylactic/ therapeutic agent for these diseases. Furthermore, compounds derived from the compound obtained by the screening described above may also be used as well.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts include salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.), and the like.

A pharmaceutical agent comprising the compound obtained by the above screening method or its salt can be manufactured in a manner similar to the method for preparing the pharmaceutical agent comprising the protein of the present invention described above.

Since the pharmaceutical preparation thus obtained is safe and low in toxicity, it can be administered to human or mammal (e.g., rat, mouse, guinea pig, rabbit, ovine, swine, bovine, equine, feline, canine, simian, etc.).

The dose of the compound or its salt may vary depending upon its action, target disease, subject to be administered, administration route, etc. For example, in the case of orally administration to an adult patient (body weight: 60 kg) with breast cancer, the compound is generally administered in a daily dose of about 0.1 to about 100 mg, preferably about 1.0 to about 50 mg and more preferably about 1.0 to about 20 mg. In the case of parenteral administration, the dose may vary depending upon subject to be administered, target disease, etc. For example, in the case of administration in the form of an injection preparation to an adult patient (body weight: 60 kg) with breast cancer, it is advantageous to intravenously administer the compound in a daily dose of about 0.01 to about 30 mg, preferably about 0.1 to about 20 mg, and more preferably about 0.1 to about 10 mg. For other animal species, the corresponding dose as converted based on the body weight of 60 kg can be administered.

### (7b) Method for screening a compound that promotes or inhibits the activity of a promoter to the DNA of the present invention

The present invention provides a method for screening a compound or its salt that promotes or inhibits the activity of a promoter to the DNA of the present invention, which comprises administering a test compound to a non-human mammal deficient in expression of the DNA of the present invention, and detecting the expression of a reporter gene.

In the screening method described above, an animal, in which the DNA of the present invention is inactivated by introducing a reporter gene and the reporter gene can be expressed under control of a promoter to the DNA of the present invention, is used as the non-human mammal deficient in expression of the DNA of the present invention, among the non-human mammals deficient in expression of the DNA of the present invention.

As the test compounds, substantially the same compounds as those given above are usable.

Examples of the reporter gene may be substantially the same as those described above. Preferably usable are, for example, used β-galactosidase (lacZ) gene, soluble alkaline phosphatase gene, luciferase gene, and the like.

Since the reporter gene is present under control of a promoter to the DNA of the present invention in the non-human mammal deficient in expression of the DNA of the present invention in which the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of in vivo expression of the protein of the present invention can be readily observed in an animal by staining with a reagent which acts as a substrate for β-galactosidase, e.g., 5-bromo-4-chloro-3-indolyl-β-galactopyranoside (X-gal). Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue sample with 1 mM EDTA/PBS solution, the color exhibited is observed. Alternatively, mRNA encoding lacZ may be detected in a conventional manner.

The compound or its salt obtained using the screening method described above is a compound selected from the test compounds described above and that promotes or inhibits the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts include salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.), and the like.

The compound or its salt promoting or inhibiting the promoter activity to the DNA of the present invention can regulate the expression of the protein of the present invention and can regulate the functions of the protein. Thus, the compound or its salt is useful, for example, as a preventive/therapeutic agent, a cell apoptosis promoter, a cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

In addition, compounds derived from the compound obtained by the screening described above may also be used as well.

A pharmaceutical agent comprising the compound obtained by the above screening method or a salt thereof can be manufactured in a manner similar to the method for preparing the pharmaceutical agent comprising the protein of the present invention described above.

Since the pharmaceutical preparation thus obtained is safe and low in toxicity, it can be administered to human or mammal (e.g., rat, mouse, guinea pig, rabbit, ovine, swine, bovine, equine, feline, canine, simian, etc.).

The dose of the compound or its salt may vary depending upon its action, target disease, subject to be administered, administration route, etc. For example, in the case of orally administration to an adult patient (body weight: 60 kg) with breast cancer, the compound inhibiting the promoter activity to the DNA of the present invention is generally administered in a daily dose of about 0.1 to about 100 mg, preferably about 1.0 to about 50 mg and more preferably about 1.0 to about 20 mg. In the case of parenteral administration, the dose may vary depending upon subject to be administered, target disease, etc. For example, in the case of administration in the form of an injection preparation to an adult patient (body weight: 60 kg) with breast cancer, it is advantageous to intravenously administer the compound inhibiting the promoter activity to the DNA of the present invention in a daily dose of about 0.01 to about 30 mg, preferably about 0.1 to about 20 mg, and more preferably about 0.1 to about 10 mg. For other animal species, the corresponding dose as converted based on the body weight of 60 kg can be administered.

As stated above, the non-human mammal deficient in expression of the DNA of the present invention is extremely useful for screening a compound or its salt that promotes or inhibits the promoter activity to the DNA of the present invention, and can greatly contribute to elucidation of causes for various diseases suspected to be caused by deficiency in expression of the DNA of the present invention and to the development of prophylactic/therapeutic agents for these diseases.

In addition, a so-called transgenic animal (gene transferred animal) can be prepared using a DNA containing the promoter region of the protein of the present invention, by ligating genes encoding various proteins at the downstream to the promoter region and injecting the same into oocyte of an animal. It is thus possible to synthesize the protein therein specifically and study the in vivo activity in the animal. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification and the sequence listing, the codes of bases, amino acids, etc. are denoted in accordance with the IUPAC-IUB Commission on Biochemical Nomenclature or by the common codes used in the art, examples of which are shown below. For amino acids that may have an optical isomer, L form is presented unless otherwise indicated.
DNA : deoxyribonucleic acid
cDNA : complementary deoxyribonucleic acid
A :adenine
T :thymine
G :guanine
C :cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
Gly : glycine
Ala : alanine
Val : valine
Leu :leucine
Ile : isoleucine
Ser :serine
Thr : threonine
Cys : cysteine
Met : methionine
Glu :glutamic acid
Asp :aspartic acid
Lys :lysine
Arg : arginine
His : histidine
Phe : phenylalanine
Tyr : tyrosine
Trp : tryptophan
Pro : proline
Asn : asparagine
Gln : glutamine
pGlu : pyroglutamic acid
Sec : selenocysteine

Substituents, protecting groups and reagents generally used in this specification are presented as the codes below.
Me : methyl group
Et : ethyl group
Bu : butyl group
Ph : phenyl group
TC : thiazolidine-4(R)-carboxamido group
Tos : p-toluenesulfonyl
CHO : formyl
Bzl : benzyl
Cl₂-Bzl : 2,6-dichlorobenzyl
Bom : benzyloxymethyl
Z : benzyloxycarbonyl
Cl-Z : 2-chlorobenzyloxycarbonyl
Br-Z : 2-bromobenzyloxycarbonyl
Boc : t-butoxycarbonyl
DNP : dinitrophenol
Trt : trityl
Bum : t-butoxymethyl
Fmoc : N-9-fluorenylmethoxycarbonyl
HOBt : 1-hydroxybenztriazole
HOOBt : 3,4-dihydro-3-hydroxy-4-oxo-1,2,3-benzotriazine
HONB :1-hydroxy-5-norbornene-2,3-dicarboxyimide
DCC: N,N'-dicyclohexylcarbodiimide

The sequence identification numbers in the sequence listing of the specification indicate the following sequence.
[SEQ ID NO: 1]
   This shows the amino acid sequence of human HHAT.
[SEQ ID NO: 2]
   This shows the nucleotide sequence of cDNA encoding the human HHAT.
[SEQ ID NO: 3]
   This shows the amino acid sequence of mouse HHAT.
[SEQ ID NO: 4]
   This shows the nucleotide sequence of cDNA encoding the mouse HHAT.
[SEQ ID NO: 5]
   This shows the amino acid sequence of drosophila skinny.
[SEQ ID NO: 6]
   This shows the nucleotide sequence of cDNA encoding the drosophila skinny.
[SEQ ID NO: 7]
   This shows the nucleotide sequence of the primer used in EXAMPLE 1 (1).
[SEQ ID NO: 8]
   This shows the nucleotide sequence of the primer used in EXAMPLE 1(1).
[SEQ ID NO: 9]
   This shows the nucleotide sequence of the primer used in EXAMPLE 1(1).
[SEQ ID NO: 10]
   This shows the nucleotide sequence of the primer used in EXAMPLE 1(1).
[SEQ ID NO: 11]
   This shows the nucleotide sequence of the primer used in EXAMPLE 1(3).
[SEQ ID NO: 12]
   This shows the nucleotide sequence of the primer used in EXAMPLE 1(3).
[SEQ ID NO: 13]
   This shows the nucleotide sequence of the primer used in EXAMPLE 1(3).
[SEQ ID NO: 14]
   This shows the nucleotide sequence of the primer used in EXAMPLE 1(3).
[SEQ ID NO: 15]
   This shows the nucleotide sequence of the primer used in EXAMPLE 1(3).
[SEQ ID NO: 16]
   This shows the nucleotide sequence of the primer used in EXAMPLE 1(3).
[SEQ ID NO: 17]
   This shows the nucleotide sequence of the primer used in EXAMPLE 1(3).
[SEQ ID NO: 18]
   This shows the nucleotide sequence of the primer used in EXAMPLE 1(3).
[SEQ ID NO: 19]
   This shows the nucleotide sequence of siRNA used in EXAMPLE 2.
[SEQ ID NO: 20]
   This shows the nucleotide sequence of siRNA used in EXAMPLE 2.
[SEQ ID NO: 21]
   This shows the nucleotide sequence of siRNA used in EXAMPLE 2.
[SEQ ID NO: 22]
   This shows the amino acid sequence of human Shh.
[SEQ ID NO: 23]
   This shows the nucleotide sequence of cDNA encoding the human Shh.

Hereinafter the present invention will be described more specifically by referring to EXAMPLES but is not deemed to be limited thereto.

### EXAMPLE 1

### (1) cDNA cloning and construction of the expression vector of human Shh

cDNA of human Shh was cloned by a PCR method based on the known sequence (NM_000193).

Two types of PCR primers (sense sequence: SEQ ID NO: 7; antisense sequence: SEQ ID NO: 8) were used. The composition of the PCR reaction solution was obtained by adding 2 µl of fetal human embryo derived Marathon ready cDNA (Clontech), 5 µl of 10 x Pfu reaction buffer (Stratagene), 1 µl of Pfu turbo polymerase (Stratagene), 1 µl of each primer (20 µM) and 4 µl of 2.5 mM dNTP mixture (Takara Bio Inc.), and making the solution amount 50 µl with distilled water. The PCR reaction was carried out by repeating 40 times the cycle of reaction at 98°C for 1 minute, at 98°C for 30 seconds, at 64°C for 30 seconds, and at 72°C for 2 minutes, and then performing an extension reaction at 72°C for 7 minutes. After the reaction was terminated, the PCR product was treated with restriction enzymes EcoRI and XbaI. The PCR product treated with the restriction enzymes was ligated with pcDNA3.1(+) treated with the restriction enzymes EcoRI and XbaI. The resultant substance was used to transform Escherichia coli DH5α. The obtained transformant strain was cultured, and then plasmid DNA was extracted. The plasmid was subjected to a sequencing reaction using the Big Dye Terminator Cycle Sequencing ver 3.1 (Applied Biosystems). Using the 3100 DNA Sequencer (Applied Biosystems), it was confirmed that the nucleotide sequence of the PCR product was matched to SEQ ID NO: 23. In this manner, cDNA containing the entire length ORF of human Shh was obtained. The main part of the human Shh providing the activity thereof is considered to be a part corresponding to the cysteine residue at the 24th position up to the glycine residue at the 197th position (hereinafter, this part will be sometimes referred to as the "human Shh-N") (Williams et al., J. Cell Sci., 112, 4405-4414, 1998). The E. coli expression vector of the human Shh-N was constructed as follows. A DNA sequence including tag formed of 6 histidines (6 x His Tag) and enterokinase cleavage site which are added to the 5'-terminus of human Shh-N was synthesized by the PCR method using two types of primers (sense sequence: SEQ ID NO: 9; antisense sequence: SEQ ID NO: 10). The composition of the PCR reaction solution was obtained by adding 10 ng of the above-mentioned plasmid containing the entire sequence of Shh, 5 µl of ExTaq buffer (Takara Bio Inc.), 0.5 µl of ExTaq polymerase (Takara Bio Inc.), 1 µl of each primer (20 µM), and 4 µl of 2.5 mM dNTP (Takara Bio Inc.), and making the solution amount 50 µl with distilled water. The PCR reaction was carried out by repeating 30 times the cycle of reaction at 98°C for 2 minutes, at 95°C for 20 seconds, at 65°C for 30 seconds, and at 72°C for 30 seconds, and then performing an extension reaction at 72°C for 5 minutes. After the reaction was terminated, the PCR product was treated with restriction enzymes NcoI and BamHI, and then ligated with pET11d (Novagen) treated in advance with the restriction enzymes NcoI and BamHI. After the ligation reaction, a transformant strain obtained by transformation of E. coli DH5α was cultured. Then, DNA was extracted, and subjected to a sequence reaction using the Big Dye Terminator Cycle Sequencing ver 3.1. Using the 3100 DNA Sequencer, it was confirmed that the nucleotide sequence inserted to the plasmid matched to the sequence of the 72nd position up to the 591 st position of the nucleotide sequence represented by SEQ ID NO: 23.

### (2) Preparation of E. coli derived human Shh recombinant protein

In order to obtain a substrate for the activity measurement of HHAT, a protein having recombinant E. coli in the N-terminal part of the human Shh was produced.

E. coli BL21 (DE3)/pLysS (Novagen) was transformed with the expression vector produced in EXAMPLE 1(1) was transformed, and the obtained transformant strain was pre-cultured in 2 ml of LB medium. Then, the seeded culture was added to 100 ml of LB medium containing 1 mM isopropyl-thio-β-D-galactopyranoside (IPTG) and cultured for 2 hours. The culture was centrifuged at 10,000 x g for 10 minutes to recover the bacteria. The bacteria was incubated in 5 ml of 50 mM Tris-HCl (pH7.5) buffer, containing the Bug Buster Protein Extraction reagent (concentrated 10 folds) (Novagen), 150 mM NaCl, 0.5 mM DTT, 1 mM PMSF, 20 mM imidazole, and 125 units of Benzonase Nuclease (Novagen), at room temperature for 20 minutes to be dissolved. The resultant bacteria was centrifuged at 16,000 x g for 20 minutes to obtain the supernatant as a cell extract. The cell extract was applied to the HisTrap HP column (Amersham) equilibrated in advance with 20 mM phosphate buffered saline (pH7.4) (hereinafter, referred to as the "connecting buffer") containing 0.5 M NaCl, 0.5 mM DTT and 20 mM imidazole. The resultant substance was washed with the connecting buffer, and then a histidine fused recombinant protein was eluted by 5 fractions (each of 1 ml) with 20 mM phosphate buffer (pH7.4) containing 0.5 M NaCl, 0.5 mM DTT and 300 mM imidazole. A fraction containing the protein was desalted with the HiTrap Desalting column (Amersham), and then enterokinase (Roche) was added thereto in an amount of 1/400 of the substrate protein (% by weight) and treated at room temperature overnight. Thus, a histidine tag was excised.

### (3) cDNA cloning and construction of the expression vector of human hedgehog acyltransferase (human HHAT) and drosophila skinny

cDNAs of human HHAT and drosophila skinny were respectively cloned by PCR reactions based on the known sequences NM_018194 and NM_079174. For cloning the human HHAT, two types of primers (sense sequence: SEQ ID NO: 11; antisense sequence: SEQ ID NO: 12) were used. The composition of the PCR reaction solution was obtained by adding 2 µl of fetal human embryo derived Marathon ready cDNA (Clontech), 5 µl of 10 x Pfu buffer, 1 µl of Pfu turbo polymerase, 1 µl of each primer (20 µM) and 4 µl of 2.5 mM dNTP, and making the solution amount 50 µl with distilled water. The PCR reaction was carried out by repeating 40 times the cycle of reaction at 98°C for 1 minute, at 98°C for 30 seconds, at 64°C for 30 seconds, and at 72°C for 5 minutes, and then performing an extension reaction at 72°C for 7 minutes. After the reaction was terminated, the PCR product was treated with restriction enzymes EcoRI and XbaI. The PCR product treated with the restriction enzymes was ligated with pcDNA3.1(+) treated with the restriction enzymes EcoRI and XbaI. The resultant substance was used to transform Escherichia coli DH5α. The obtained transformant strain was cultured, and then plasmid DNA was extracted.

For cloning the drosophila skinny, two types of primers (sense sequence: SEQ ID NO: 13; antisense sequence: SEQ ID NO: 14) were used. Drosophila embryo derived cDNA (Clontech) was used as a template and amplified. The composition of the PCR reaction solution was obtained by adding 0.5 µl of drosophila embryo derived cDNA (Clontech), 2 µl of Pfu buffer, 0.5 µl of Hot start Pfu, 1 µl of each primer (20 µM) and 1.6 µl of 2.5 mM dNTP, and making the solution amount 20 µl with distilled water. The PCR reaction was carried out by repeating 32 times the cycle of reaction at 95°C for 2 minutes, at 95°C for 20 seconds, at 60°C for 30 seconds, and at 72°C for 2 minutes, and then performing an extension reaction at 72°C for 10 minutes. Ex-Taq was used to add A to the 3'-terminus of the obtained product, and the obtained substance was ligated to pT7Blue. This was used to transform Escherichia coli DH5α. The obtained transformant strain was cultured, and then plasmid DNA was extracted.

Each obtained plasmid was subjected to a sequence reaction using the Big Dye Terminator Cycle Sequencing ver 3.1 (Applied Biosystems). Using the 3100 DNA Sequencer (Applied Biosystems), it was confirmed that the nucleotide sequences inserted to the plasmids were respectively matched to human HHAT cDNA (SEQ ID NO: 2) and drosophila skinny cDNA.

An animal cell expression vector with a C-terminal FLAG-Tag of the human HHAT or the drosophila skinny was produced by amplifying the sequence obtained by removing the termination codon from the entire ORF of the human HHAT or the drosophila skinny, and inserting the amplified sequence into pFLAG-CMV5a.

The composition of the PCR reaction solution for constructing a human HHAT expression vector was obtained by adding 10 ng of the above-mentioned plasmid containing the human HHAT cDNA, 5 µl of 10 Pfu reaction buffer, 1 µl of Hot start Pfu polymerase (Stratagene), 1 µl of each primer (20 µM) (SEQ ID NO: 15 and SEQ ID NO: 16) and 4 µl of 2.5 mM dNTP, and making the solution amount 50 µl with distilled water. The PCR reaction was carried out by repeating 30 times the cycle of reaction at 95°C for 2 minutes, at 95°C for 20 seconds, at 62°C for 30 seconds, and at 72°C for 2 minutes, and then performing an extension reaction at 72°C for 10 minutes. The PCR product was treated with restriction enzymes EcoRI and KpnI, and then ligated to pFLAG-CMV5a. The ligation reaction product was used to transform E. coli DH5α. The obtained transformant strain was cultured, and then plasmid DNA was extracted.

A drosophila skinny expression vector was constructed as follows. Using primers (SEQ ID NO: 17 and SEQ ID NO: 18), PCR was carried out in the same conditions as for constructing the human HHAT using 10 ng of the above-mentioned plasmid containing the drosophila skinny cDNA. The obtained PCR product was treated with restriction enzymes NotI and KpnI, and then ligated to pFLAG-CMV5a.

The plasmid DNA of each expression vector was subjected to a sequence reaction using the Big Dye Terminator Cycle Sequencing ver 3.1. Using the 3100 DNA Sequencer, it was confirmed that the nucleotide sequences inserted to the plasmids were respectively matched to human HHAT cDNA (SEQ ID NO: 2) and drosophila skinny cDNA.

### (4) Preparation of crude enzyme extract for HHAT activity measurement

HEK293 cells (purchased from Dainippon Seiyaku) were cultured in 10% fetal bovine serum-containing DMEM medium, and planted to a 6-well Type-I collagen coated plate (Iwaki) at a cell density of 4 × 10⁵ cells/well on the day before gene introduction. After being washed with a serum-free DMEM medium, 1 µg of each of the human HHAT expression vector and the skinny expression vector produced in EXAMPLE 1(2) and pcDNA3.1(+) as a control was added to 2 ml of OPTI MEM-I medium (Invitrogen) together with 8 µl of lipofectoamine (Qiagen) and 6 µl of PLUS reagent (Qiagen), and then gene introduction was carried out. The cell to which gene had been introduced were cultured under 5% CO₂ at 37°C. 48 hours later, the cells were washed twice with PBS, and recovered with 200 µl of Tris-HCl (pH7.0) buffer containing 250 mM sucrose, 1 mM EDTA and a protease inhibitor cocktail (Roche). The cells were crushed with sonication to prepare a Human HHAT crude enzyme solution and a skinny crude enzyme solution.

### (5) Measurement of HHAT activity

To a reaction buffer having a composition of 150 mM NaCl, 1 mM EDTA and 20 mM Tris-HCl (pH7.4), human Shh-N having a final concentration of 75 µg/ml, Coenzyme A having a final concentration of 50 ng/ml (Sigma), 0.3 mM ATP (Roche), 1 µCi[¹⁴C] palmitic acid (Daiichi Pure Chemicals Co., Ltd.), and the HHAT crude enzyme solution obtained in section (4) above in a volume of 1/4 of the total volume were added to prepare an enzyme reaction solution. 40 µl of the enzyme reaction solution was incubated at room temperature for 1 hour. 13 µl of the incubated reaction solution was subjected to polyacrylamide gel electrophoresis (10% acrylamide, reducing conditions, constant voltage: 200 V) using the NuPAGE system (Invitrogen) in accordance with the method described in the attached sheet. Before the electrophoresis, the sample was heat-treated at 70°C for 10 minutes. The gel obtained after the electrophoresis was exposed to light on the BAS imaging plate overnight to analyze the image using the BAS2000 imaging analyzer (Fujifilm) and thus to quantify the radioactivity of the band. Only when the human HHAT crude enzyme solution and the Shh-N were reacted, a band having a radioactivity was recognized in the vicinity of the molecular weight of 21 kDa corresponding to the Shh-N. With the crude enzyme solution fraction extracted from HEK293 cells to which pcDNA3.1 or the skinny expression vector was introduced by the same method as for the human HHAT, no band having a radioactivity was recognized.

Fig. 1 shows the results of quantification of the radioactivity of the bands recognized with electrophoresis.

The quantified value for the human HHAT is the value obtained when palmitic acid was added to Shh.

### EXAMPLE 2

### Effect of suppressing cancer cell proliferation by HHAT gene knockdown

HT-29 cells from the cell line derived from human E. coli were planted to a 96-well plate at a density of 2.5 × 10³ cells/well. After culturing the cells overnight, siRNA to human Shh (Ambion; SEQ ID NO: 19), siRNA to human HHAT (Ambion; SEQ ID NO: 20), or non-silencing siRNA (Dhamacon, SEQ ID NO: 21) as a control was introduced. The introduction was performed by adding a mixture of 0.4 µl of DhamaFECT4 reagent (Dhamacon) per well and 100 nM of each siRNA to the cells. The knockdown efficiency of each siRNA at this point was checked with the TaqMan method. On the 5th day after the introduction of siRNA, total RNA was extracted from the HT-29 cells using RNeasy mini kit (QIAGEN), and cDNA was synthesized from a random primer using the TaqMan Gold RT-PCR kit (Applied Biosystems). 2 µl of the synthesized cDNA, 10 µl of 2 × TaqMan Universal PCR Master Mix (Applied Biosystems), 1 µl of 20 × TaqMan Gene Expression Assay (Applied Biosystems) to the human Shh or HHAT, and 7 µl of distilled water were added together to a total amount of 20 µl. This was used as a reaction solution of quantificative RT-PCR.

Regarding the reaction solution, the expression of human Shh or human HHAT was quantified in the ratio to GAPDH using the ABI PRISM 7700 Sequence Detection System. As compared with the control, the gene expression was suppressed by about 75% in the case of siRNA to human Shh and by about 60% in the case of siRNA to human HHAT.

In order to quantify the cell proliferation suppression at this point, the number of cells was measured using CellTiter-Glo Luminescent Cell Viability Assay (Promega) 5 days after the addition of each siRNA to the HT-29 cells.

As a result, as compared to the cells supplemented with the non-silencing siRNA, the cells supplemented with the siRNA to human Shh or the siRNA to human HHAT exhibited the proliferation inhibition of about 50 to 60%.

The proliferation inhibition activity provided by the knockdown of the human HHAT gene was approximately equivalent to the effect provided by the knockdown of the human Shh gene. This suggests that the knockdown of the HHAT gene suppresses the function of Shh almost completely.

### EXAMPLE 3

### Method for searching for human HHAT inhibiting compound using radiolabeled palmitic acid

To 10 µM human Shh-N, or partial peptide including the amino acids from the 24th position up to the 37th position of human Shh, both modified with biotin in advance, 20 µM [³H] palmitoyl CoA, a test compound having a predetermined concentration, and a HHAT crude enzyme solution are added, and a reaction is carried out at room temperature in a reaction buffer (150 mM NaCl, 1 mM EDTA and 20 mM Tris-HCl (ph7.4)). To the reaction solution, streptavidin-conjugated SPA beads (Amersham) are added to bond the human Shh-N protein or the N-terminal peptide of human Shh to the beads. The radioactivity derived from the palmitic acid added to the N-terminus of human Shh increases depending on the HHAT activity. This is measured by a scintillation counter. The reduction rate of the HHAT activity caused by the addition of the test compound, with respect to the value with no addition of the test compound, is calculated, and thus a compound having the HHAT inhibiting function is identified.

### EXAMPLE 4

### Method for searching for inhibiting compound using HHAT expressing cells

As compared with the protein or peptide which is not modified with lipid, a protein or peptide which is modified with lipid remains in the cell or on the cell surface at a higher possibility. This property can be used to search for a compound having an HHAT inhibiting action.

Animal cells expressing HHAT at a high level are produced and cultured on a 96-well plate for 24 hours. Shh-N, or a partial peptide comprised of amino acids from the 24th position up to the 37th position of human Shh, radiolabeled in advance is added to the medium and incorporated into the cells. Next, a test compound is added and incubated for a predetermined period, and then the medium is removed. The cells are then washed 5 times with PBS containing 3.5% BSA (hereinafter, referred to simply as the "washing buffer") and then incubated at 37°C for 15 minutes in the presence of the washing buffer. In order to correct the variance of the cell density in each well, the nuclei are dyed with DRAQ5 (Biostatus). The fluorescent intensity derived from the Shh-N or the N-terminal partial peptide of Shh, and the cell density quantified with DRAQ5, are measured for each well with Discovery-I (Molecular Devices), and the obtained ratio is used to calculate the amount of the Shh protein or the N-terminal peptide of Shh remaining in the cells. Using the value obtained in the experiment performed with no test compound as a control, the HHAT activity inhibiting ratio caused by the test compound is calculated, and thus a compound having the HHAT inhibiting function is identified.

### EXAMPLE 5

### Method for searching for human HHAT inhibiting compound using radiolabeled palmitic acid

A peptide (Shh-(24-38)) having 15-mer peptide comprised of amino acids from the 24th position to the 38th position of human Shh (SEQ ID NO: 22) modified with biotin at the lysine residue at the carboxylic terminus, and a peptide (Shh-(24-45)) having 22-mer peptide comprised of amino acids from the 24th position to the 45th position of human Shh modified with biotin at the lysine residue at the carboxylic terminus, were synthesized as a substrate peptide. An HHAT activity measuring system was produced by adding 50 µl reaction buffer (10 mM Tris-HCl (pH7.4), 50 mM magnesium chloride, 2 µM [³H] palmitoyl CoA, and 400 µg/ml of HHAT enzyme solution) to a 96-well plate coated with streptavidin and a scintillator (Streptavidin FlashPlate, PerkinElmer). The above-mentioned synthetic peptides were added to the reaction system and reacted for a predetermined period. Then, the reaction was terminated by adding 200 µl of 1% aqueous solution of Triton X-100 to the reaction solution. After the reaction, the radioactivity of the reaction solution was measured by the scintillation counter as an HHAT activity.

[³H] palmitoyl CoA was prepared from [³H] palmitic acid (GE Healthcare) by a method described in Methods in Enzymology, 250, 299-300, 1995. The HHAT enzyme solution was prepared by expressing human HHAT with the Bac-to-Bac system (Invitrogen) which is a commercially available baculovirus expression system in accordance with the method described in the attached sheet.

10 µM of the substance peptide Shh-(24-38) or Shh-(24-45) was used to measure the HHAT activity when the reaction was carried out for 1 hour by the above-described activity measurement method (Fig. 2). As compared with a control experiment without using the HHAT enzyme solution, the experiment using HHAT enzyme solution exhibited a conspicuous increase of the radioactivity. Thus, it was confirmed that these peptides act as a substance in the above-described activity measurement system. The concentration of the substrate peptide was checked using Shh-(24-45) with the reaction time being fixed to 1 hour. As a result, the activity was increased depending on the concentration (Fig. 3). The reaction was checked using Shh-(24-45) with the concentration of the substrate peptide being fixed to 10 µM. As a result, the activity was increased for 2 hours (Fig. 4).

By calculating the reduction rate of the HHAT activity caused by the addition of a test compound, with respect to the value obtained with no addition of the test compound, screening of a compound having the HHAT inhibiting function can be realized with a high throughput.

### INDUSTRIAL APPLICABILITY

A protein comprising the same or substantially the same amino acid sequence as the amino acid sequence represented by SEQ ID NO: 1 (the protein used in the present invention) has an acyltransferase activity of adding lipid to the N-terminus (e.g., Cys at the 24th position of the amino acid sequence represented by SEQ ID NO: 22) of Shh (e.g., human Shh, mouse Shh, etc.) which is increasingly expressed in cancer tissues. Therefore, a compound or its salt that inhibits an activity of the protein of such a protein, a compound or its salt that inhibits the expression of a gene for the protein, an antibody to the protein, an antisense polynucleotide to a polynucleotide encoding the protein, siRNA, shRNA, and the like are usable as a cancer preventive/therapeutic agent (including a cancer metastasis suppressing agent), a cancer cell apoptosis promoter, a cancer cell proliferation suppressor or the like for cancers (e.g., brain tumor, medulloblastoma, glioma, pituitary tumor, neuroglia, acoustic neuroma, pharyngeal cancer, laryngeal cancer, tongue cancer, thymoma, mesothelioma, breast cancer, lung cancer, non-small cell lung cancer, small cell lung cancer, gastric cancer, esophageal cancer, colorectal cancer, colon cancer, rectal cancer, liver cancer, hepatocellular carcinoma, pancreatic cancer, pancreatic endocrine tumor, bile duct cancer, gallbladder cancer, penile cancer, renal cancer, renal pelvic cancer, urethral cancer, renal cell cancer, testicular tumor, prostate cancer, bladder cancer, vulvar cancer, uterine cancer, cervical cancer, uterine body cancer, uterine sarcoma, trophoblastic disease, vaginal cancer, ovary cancer, ovarian germ cell tumor, skin cancer, malignant melanoma, mycosis fungoides, basalioma, soft part sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, unknown primary cancer, etc.; preferably, medulloblastoma, breast cancer, small cell lung cancer, gastric cancer, esophageal cancer, colorectal cancer, colon cancer, pancreatic cancer, bile duct cancer, prostate cancer, etc.).

## Claims

1. A prophylactic/therapeutic agent for cancer, comprising a compound or its salt that inhibits an activity of a protein comprising the same or substantially the same amino acid sequence as the amino acid sequence represented by SEQ ID NO: 1, its partial peptide, or a salt thereof.

2. A prophylactic/therapeutic agent for cancer, comprising a compound or its salt that inhibits the expression of a gene for a protein comprising the same or substantially the same amino acid sequence as the amino acid sequence represented by SEQ ID NO: 1, its partial peptide, or a salt thereof.

3. An antisense polynucleotide, comprising the entirety or part of a nucleotide sequence complementary or substantially complementary to a nucleotide sequence of a polynucleotide encoding a protein having the same or substantially the same amino acid sequence as the amino acid sequence represented by SEQ ID NO: 1, or its partial peptide.

4. A pharmaceutical agent, comprising the antisense polynucleotide according to claim 3.

5. The pharmaceutical agent according to claim 4, which is a prophylactic/therapeutic agent for cancer.

6. A pharmaceutical agent, comprising an siRNA or an shRNA against a polynucleotide encoding a protein having the same or substantially the same amino acid sequence as the amino acid sequence represented by SEQ ID NO: 1, or its partial peptide.

7. The pharmaceutical agent according to claim 6, which is a prophylactic/therapeutic agent for cancer.

8. An antibody against a protein having the same or substantially the same amino acid sequence as the amino acid sequence represented by SEQ ID NO: 1, its partial peptide, or a salt thereof.

9. A prophylactic/therapeutic agent for cancer, comprising the antibody according to claim 8.

10. A diagnostic agent for cancer, comprising the antibody according to claim 8.

11. A diagnostic agent for cancer, comprising a polynucleotide encoding a protein having the same or substantially the same amino acid sequence as the amino acid sequence represented by SEQ ID NO: 1, or its partial peptide.

12. A method for diagnosing cancer, which comprises using the antibody according to claim 8 or a polynucleotide encoding a protein having the same or substantially the same amino acid sequence as the amino acid sequence represented by SEQ ID NO: 1, or its partial peptide.

13. Use of a protein having the same or substantially the same amino acid sequence as the amino acid sequence represented by SEQ ID NO: 1, its partial peptide, or a salt thereof, as a diagnostic marker for cancer.

14. A method for screening a prophylactic/therapeutic pharmaceutical agent for cancer, which comprises using a protein having the same or substantially the same amino acid sequence as the amino acid sequence represented by SEQ ID NO: 1, its partial peptide, or a salt thereof.

15. A kit for screening a prophylactic/therapeutic pharmaceutical agent for cancer, comprising a protein having the same or substantially the same amino acid sequence as the amino acid sequence represented by SEQ ID NO: 1, its partial peptide, or a salt thereof.

16. A method for screening a prophylactic/therapeutic pharmaceutical agent for cancer, which comprises using a polynucleotide encoding a protein having the same or substantially the same amino acid sequence as the amino acid sequence represented by SEQ ID NO: 1, or its partial peptide.

17. A kit for screening a prophylactic/therapeutic pharmaceutical agent for cancer, comprising a polynucleotide encoding a protein having the same or substantially the same amino acid sequence as the amino acid sequence represented by SEQ ID NO: 1, or its partial peptide.

18. A method for screening a prophylactic/therapeutic pharmaceutical agent for cancer, which comprises measuring an activity of a protein having the same or substantially the same amino acid sequence as the amino acid sequence represented by SEQ ID NO: 1, its partial peptide, or a salt thereof.

19. A method for screening a prophylactic/therapeutic pharmaceutical agent for cancer, which comprises measuring an amount of a protein having the same or substantially the same amino acid sequence as the amino acid sequence represented by SEQ ID NO: 1, its partial peptide, or a salt thereof.

20. A method for preventing/treating cancer, which comprises inhibiting an activity of a protein having the same or substantially the same amino acid sequence as the amino acid sequence represented by SEQ ID NO: 1, its partial peptide, or a salt thereof, or inhibiting the expression of a gene for the protein, its partial peptide, or a salt thereof.

21. A method for preventing/treating cancer, which comprises administering to a mammal an effective dose of (i) a compound or its salt that inhibits an activity of a protein having the same or substantially the same amino acid sequence as the amino acid sequence represented by SEQ ID NO: 1, its partial peptide, or a salt thereof, (ii) a compound or its salt that inhibits the expression of a gene for the protein, its partial peptide, or a salt thereof, (iii) the antibody according to claim 8, or (iv) an antisense polynucleotide comprising the entirety or part of a nucleotide sequence complementary or substantially complementary to a nucleotide sequence of a polynucleotide encoding the protein or its partial peptide.

22. Use of (i) a compound or its salt that inhibits an activity of a protein having the same or substantially the same amino acid sequence as the amino acid sequence represented by SEQ ID NO: 1, its partial peptide, or a salt thereof, (ii) a compound or its salt that inhibits the expression of a gene for the protein, its partial peptide, or a salt thereof, (iii) the antibody according to claim 8, or (iv) an antisense polynucleotide comprising the entirety or part of a nucleotide sequence complementary or substantially complementary to a nucleotide sequence of a polynucleotide encoding the protein or its partial peptide, for the manufacture of a prophylactic/therapeutic agent for cancer,.

23. An agent for promoting apoptosis of cancer cells or an agent for suppressing proliferation of cancer cells, comprising a compound or its salt that inhibits an activity of a protein comprising the same or substantially the same amino acid sequence as the amino acid sequence represented by SEQ ID NO: 1, its partial peptide, or a salt thereof.

24. An agent for promoting apoptosis of cancer cells or an agent for suppressing proliferation of cancer cells, comprising a compound or its salt that inhibits the expression of a gene for a protein comprising the same or substantially the same amino acid sequence as the amino acid sequence represented by SEQ ID NO: 1, its partial peptide, or a salt thereof.

25. The pharmaceutical agent according to claim 4, which is an agent for promoting apoptosis of a cancer cell or suppressing proliferation of a cancer cell.

26. The pharmaceutical agent according to claim 6, which is an agent for promoting apoptosis of a cancer cell or suppressing proliferation of a cancer cell.

27. An agent for promoting apoptosis of a cancer cell or suppressing proliferation of a cancer cell, comprising the antibody according to claim 8.

28. A method for screening a pharmaceutical agent for promoting apoptosis of a cancer cell or suppressing proliferation of a cancer cell, which comprises using a protein having the same or substantially the same amino acid sequence as the amino acid sequence represented by SEQ ID NO: 1, its partial peptide, or a salt thereof.

29. A kit for screening a pharmaceutical agent for promoting apoptosis of a cancer cell or suppressing proliferation of a cancer cell, comprising a protein having the same or substantially the same amino acid sequence as the amino acid sequence represented by SEQ ID NO: 1, its partial peptide, or a salt thereof.

30. A method for screening a pharmaceutical agent for promoting apoptosis of a cancer cell or suppressing proliferation of a cancer cell, which comprises using a polynucleotide encoding a protein having the same or substantially the same amino acid sequence as the amino acid sequence represented by SEQ ID NO: 1, or its partial peptide.

31. A kit for screening a pharmaceutical agent for promoting apoptosis of a cancer cell or suppressing proliferation of a cancer cell, comprising a polynucleotide encoding a protein having the same or substantially the same amino acid sequence as the amino acid sequence represented by SEQ ID NO: 1, or its partial peptide.

32. A method for promoting apoptosis of a cancer cell or suppressing proliferation of a cancer cell, which comprises inhibiting an activity of a protein having the same or substantially the same amino acid sequence as the amino acid sequence represented by SEQ ID NO: 1, its partial peptide, or a salt thereof, or inhibiting the expression of a gene for the protein, its partial peptide, or a salt thereof.

33. A method for promoting apoptosis of a cancer cell or suppressing proliferation of a cancer cell, which comprises administering to a mammal an effective dose of (i) a compound or its salt that inhibits an activity of a protein having the same or substantially the same amino acid sequence as the amino acid sequence represented by SEQ ID NO: 1, its partial peptide, or a salt thereof, (ii) a compound or its salt that inhibits the expression of a gene for the protein, its partial peptide or a salt thereof, (iii) an antibody against the protein, its partial peptide, or a salt thereof, or (iv) an antisense polynucleotide comprising the entirety or part of a nucleotide sequence complementary or substantially complementary to a nucleotide sequence of a polynucleotide encoding the protein or its partial peptide.

34. Use of (i) a compound or its salt that inhibits an activity of a protein having the same or substantially the same amino acid sequence as the amino acid sequence represented by SEQ ID NO: 1, its partial peptide, or a salt thereof, (ii) a compound or its salt that inhibits the expression of a gene for the protein, its partial peptide, or a salt thereof, (iii) an antibody against the protein, its partial peptide, or a salt thereof, or (iv) an antisense polynucleotide comprising the entirety or part of a nucleotide sequence complementary or substantially complementary to a nucleotide sequence of a polynucleotide encoding the protein or its partial peptide, for the manufacture of an agent for promoting apoptosis of a cancer cell or suppressing proliferation of a cancer cell.


SEQUENCE LISTING

<110> Takeda Pharmaceutical Company Limited

<120> Prophylactic/therapeutic agent for cancer

<130> G07-0060

<140> PCT/JP2006/306784
<141> 2006-03-24

<150> JP2005-89613
<151> 2005-03-25

<160> 23

<210> 1
<211> 493
<212> PRT
<213> Homo sapience

<400> 1

Met Pro Pro Arg Trp Glu Leu Ala Leu Tyr Leu Leu Ala Ser Leu Gly
                5                   10                  15
Phe His Phe Tyr Ser Phe Tyr Glu Val Tyr Lys Val Ser Arg Glu His
            20                  25                  30
Glu Glu Glu Leu Asp Gln Glu Phe Glu Leu Glu Thr Asp Thr Leu Phe
        35                  40                  45
Gly Gly Leu Lys Lys Asp Ala Thr Asp Phe Glu Trp Ser Phe Trp Met
    50                  55                  60
Glu Trp Gly Lys Gln Trp Leu Val Trp Leu Leu Leu Gly His Met Val
65                  70                  75                  80
Val Ser Gln Met Ala Thr Leu Leu Ala Arg Lys His Arg Pro Trp Ile
                85                  90                  95
Leu Met Leu Tyr Gly Met Trp Ala Cys Trp Cys Val Leu Gly Thr Pro

            100                 105                 110
Gly Val Ala Met Val Leu Leu His Thr Thr Ile Ser Phe Cys Val Ala
        115                 120                 125
Gln Phe Arg Ser Gln Leu Leu Thr Trp Leu Cys Ser Leu Leu Leu Leu
    130                 135                 140
Ser Thr Leu Arg Leu Gln Gly Val Glu Glu Val Lys Arg Arg Trp Tyr
145                 150                 155                 160
Lys Thr Glu Asn Glu Tyr Tyr Leu Leu Gln Phe Thr Leu Thr Val Arg
                165                 170                 175
Cys Leu Tyr Tyr Thr Ser Phe Ser Leu Glu Leu Cys Trp Gln Gln Leu
            180                 185                 190
Pro Ala Ala Ser Thr Ser Tyr Ser Phe Pro Trp Met Leu Ala Tyr Val
        195                 200                 205
Phe Tyr Tyr Pro Val Leu His Asn Gly Pro Ile Leu Ser Phe Ser Glu
    210                 215                 220
Phe Ile Lys Gln Met Gln Gln Gln Glu His Asp Ser Leu Lys Ala Ser
225                 230                 235                 240
Leu Cys Val Leu Ala Leu Gly Leu Gly Arg Leu Leu Cys Trp Trp Trp
                245                 250                 255
Leu Ala Glu Leu Met Ala His Leu Met Tyr Met His Ala Ile Tyr Ser
            260                 265                 270
Ser Ile Pro Leu Leu Glu Thr Val Ser Cys Trp Thr Leu Gly Gly Leu
        275                 280                 285
Ala Leu Ala Gln Val Leu Phe Phe Tyr Val Lys Tyr Leu Val Leu Phe
    290                 295                 300
Gly Val Pro Ala Leu Leu Met Arg Leu Asp Gly Leu Thr Pro Pro Ala
305                 310                 315                 320
Leu Pro Arg Cys Val Ser Thr Met Phe Ser Phe Thr Gly Met Trp Arg
                325                 330                 335
Tyr Phe Asp Val Gly Leu His Asn Phe Leu Ile Arg Tyr Val Tyr Ile
            340                 345                 350
Pro Val Gly Gly Ser Gln His Gly Leu Leu Gly Thr Leu Phe Ser Thr
        355                 360                 365
Ala Met Thr Phe Ala Phe Val Ser Tyr Trp His Gly Gly Tyr Asp Tyr
    370                 375                 380
Leu Trp Cys Trp Ala Ala Leu Asn Trp Leu Gly Val Thr Val Glu Asn

385                 390                 395                 400
Gly Val Arg Arg Leu Val Glu Thr Pro Cys Ile Gln Asp Ser Leu Ala
                405                 410                 415
Arg Tyr Phe Ser Pro Gln Ala Arg Arg Arg Phe His Ala Ala Leu Ala
            420                 425                 430
Ser Cys Ser Thr Ser Met Leu Ile Leu Ser Asn Leu Val Phe Leu Gly
        435                 440                 445
Gly Asp Glu Val Gly Lys Thr Tyr Trp Asn Arg Ile Phe Ile Gln Gly
    450                 455                 460
Trp Pro Trp Val Thr Leu Ser Val Leu Gly Phe Leu Tyr Cys Tyr Ser
465                 470                 475                 480
His Val Gly Ile Ala Trp Ala Gln Thr Tyr Ala Thr Asp
                485                 490

<210> 2
<211> 1479
<212> PRT
<213> Homo sapience

<400> 2

atgccgcccc gatgggaact ggcactttac ctacttgcct cactaggctt ccacttctat      60
tccttctatg aagtttacaa agtctccaga gaacacgaag aggagctgga ccaggaattt     120
gagctggaga ctgacacttt atttggagga ttaaagaagg atgcgaccga ctttgagtgg     180
agcttctgga tggaatgggg gaagcagtgg ctggtgtggc ttctccttgg ccacatggta     240
gtgtctcaaa tggccacact gctggcaaga aagcacagac cctggattct catgctctat     300
gggatgtggg cctgctggtg tgtgctgggg acccctggtg tggctatggt tttgctccat     360
accaccatct ctttctgcgt ggcccagttc cggtctcagc tcctgacgtg gctctgttct     420
ctcctcctcc tctccacact gaggctgcag ggtgtggaag aagttaagag aaggtggtac     480
aagacagaaa acgagtacta cctgctgcag ttcacgctga ccgttcgctg cctctactac     540
accagcttca gcctggagct ctgctggcag cagctgcctg ctgcatcgac ctcctactcc     600
tttccctgga tgctggccta tgtcttttat tatccagtct tacacaatgg gcccatcctc     660
agcttctcgg agttcatcaa acagatgcag cagcaggagc atgactccct gaaggccagc     720
ctgtgtgtcc tggccctggg gctgggccgc cttctttgct ggtggtggct ggccgagctg     780
atggctcacc tgatgtacat gcatgccatc tacagcagca tccccctcct ggagactgtc     840
tcttgttgga ccttaggagg actggcgtta gcccaggtgc tctttttcta cgtgaagtac     900

ttggtgctct ttggcgtgcc tgctctgctc atgcgcctgg atggactcac tccacccgcc 960
ctcccccgct gcgtgagcac catgttcagt ttcaccggga tgtggaggta ttttgatgtt 1020
ggactgcata atttcttaat caggtatgtg tacattccag tgggcgggtc ccagcatggc 1080
ctgctgggga cactgttttc cacggcgatg acatttgcat ttgtgagcta ctggcatggc 1140
ggctacgact acctctggtg ctgggcagcg ctcaactggc tgggagtcac tgtggagaat 1200
ggagtccgga ggctggtgga gactccctgc atccaggaca gtctggcccg atacttctcc 1260
ccacaagctc gccgtcgatt ccacgctgcc cttgcttctt gttccacctc gatgctgatc 1320
ctgtccaacc tggtatttct tgggggcaat gaggttggga aaacctactg gaataggatc 1380
ttcatacaag gctggccttg ggtgaccctc tctgtcctgg gattcctgta ctgctactcc 1440
cacgtgggca ttgcctgggc ccagacctac gccacggac 1479

<210> 3
<211> 473
<212> PRT
<213> Mus musculus

<400> 3

Met Leu Pro Gly Trp Glu Leu Thr Leu Cys Leu Leu Val Ser Leu Gly
                5                   10                  15
Phe His Phe Arg Ser Phe Tyr Glu Val Tyr Lys Val Ser Arg Glu His
            20                  25                  30
Glu Glu Glu Leu Asp Gln Glu Phe Glu Leu Glu Met Asp Thr Leu Phe
        35                  40                  45
Gly Gly Leu Lys Lys Asp Pro Thr Asp Phe Glu Trp Asn Phe Trp Met
    50                  55                  60
Glu Trp Gly Lys Arg Arg Leu Val Trp Leu Phe Ile Gly His Met Ala
65                  70                  75                  80
Val Ser Gln Leu Ala Thr Leu Leu Thr Lys Lys His Arg Pro Trp Ile
                85                  90                  95
Val Met Val Tyr Gly Met Trp Ala Cys Trp Cys Val Leu Gly Ala Pro
            100                 105                 110
Gly Val Val Met Val Leu Leu His Ser Thr Ile Ala Phe Cys Val Ala
        115                 120                 125
Gln Phe Arg Ser Val Leu Leu Ser Trp Leu Cys Ser Leu Leu Leu Leu
    130                 135                 140

Ser Thr Leu Arg Leu Gln Ser Val Glu Glu Val Lys Arg Arg Trp Tyr
145                 150                 155                 160
Lys Thr Glu Asn Glu Tyr Tyr Leu Leu Gln Phe Thr Leu Thr Val Arg
                165                 170                 175
Cys Leu Tyr Tyr Thr Ser Phe Ser Leu Glu Leu Cys Arg Gln Pro Pro
            180                 185                 190
Ser Ala Gln Pro Thr Pro Ser Ala Gln Gly Ala Ser His Ser Tyr Pro
        195                 200                 205
Trp Leu Leu Thr Tyr Val Phe Tyr Tyr Pro Val Phe His Asn Gly Pro
    210                 215                 220
Ile Leu Asn Phe Pro Glu Phe Phe Arg Gln Met Gln Gln Pro Glu Leu
225                 230                 235                 240
Asn Ser Leu Gln His Ser Leu Cys Ile Val Ala Lys Gly Leu Gly Arg
                245                 250                 255
Leu Leu Cys Trp Trp Trp Leu Ala Glu Leu Met Val His Leu Met Tyr
            260                 265                 270
Met His Ala Leu Tyr Ser Ser Ala Pro Leu Leu Glu Ser Val Ser Cys
        275                 280                 285
Trp Thr Leu Gly Gly Leu Ala Leu Ala Gln Val Leu Phe Phe Tyr Val
    290                 295                 300
Lys Tyr Leu Val Leu Phe Gly Val Pro Ala Leu Leu Met Arg Leu Asp
305                 310                 315                 320
Gly Leu Thr Pro Pro Pro Leu Pro Arg Cys Val Ser Thr Met Phe Ser
                325                 330                 335
Phe Thr Gly Met Trp Arg Tyr Phe Asp Val Gly Leu His Asn Phe Leu
            340                 345                 350
Ile Arg Tyr Val Tyr Ile Pro Leu Gly Gly Ser Gln His Gly Leu Leu
        355                 360                 365
Gly Thr Leu Leu Ser Thr Ala Thr Thr Phe Ala Phe Val Ser Tyr Trp
    370                 375                 380
His Gly Ser Tyr Glu Asp Leu Trp Cys Trp Ala Ala Leu Asn Trp Leu
385                 390                 395                 400
Gly Val Thr Val Glu Ser Gly Val Arg Arg Leu Leu Glu Thr Pro Cys
                405                 410                 415
Val Arg Glu Thr Leu Ala Arg His Leu Ser Pro Gln Ala His His Arg
            420                 425                 430

Leu His Ala Leu Leu Ala Ala Cys Ser Thr Ser Met Leu Ile Leu Phe
        435                 440                 445
Asn Leu Val Phe Leu Gly Gly Ile Gln Val Gly Lys Thr Tyr Trp Asn
    450                 455                 460
Arg Ile Phe Leu Gln Ala Ala Phe Arg
465                 470

<210> 4
<211> 1419
<212> PRT
<213> Mus musculus

<400> 4

atgctgcccg ggtgggaact gactctctgc ctgcttgttt ccttgggttt ccacttccgc      60
tctttctatg aagtttacaa agtctccaga gagcatgaag aggaactgga tcaagaattt     120
gagctggaga tggacactct atttggagga ctgaagaagg acccgactga ttttgagtgg     180
aacttctgga tggaatgggg gaagcggagg ctggtgtggc tcttcattgg ccatatggct     240
gtgtctcagt tggccacgtt actcacaaag aagcacagac cctggattgt catggtctat     300
ggaatgtggg cctgctggtg tgtgctgggg gcgccaggcg tggtcatggt ccttctccac     360
agcaccatcg cgttctgcgt ggctcagttc cgatccgtgc tcctgtcctg gctgtgttct     420
ctccttctgc tctccaccct aaggctgcaa agtgtggagg aagttaagag aaggtggtac     480
aagacagaga acgagtatta cctgctccag tttacgctca ctgttcgctg cctgtactac     540
accagcttca gcctggagct gtgccggcag cctccgtctg cacaacctac tccatctgcg     600
caaggtgcct cccattccta cccgtggctg ctgacctacg tcttctatta tcccgtcttc     660
cacaacgggc ccatccttaa cttcccggag ttcttcagac agatgcagca gccagagctc     720
aactctctgc agcacagcct ctgcattgta gccaagggcc tgggtcgtct gctctgctgg     780
tggtggctgg ctgaactgat ggtacacctc atgtacatgc atgccctcta cagcagcgcc     840
cccctcctgg agagcgtttc ctgctggacc ttaggaggac tggcgctggc ccaagtgctc     900
ttcttctatg tgaagtacct ggtgctcttt ggtgtcccgg ctctgctcat gcgcctggat     960
ggactcacgc caccacctct tccccgctgc gtgagcacca tgttcagttt taccgggatg    1020
tggaggtatt ttgatgttgg actacacaat ttcttaatca ggtatgtgta catcccactg    1080
ggcgggtccc agcatggcct gttggggaca ctgctttcta ctgcaacaac gtttgcgttc    1140
gtgagctact ggcatggtag ctatgaggac ctctggtgct gggcagcgct caactggctg    1200
ggagtcactg tggagagtgg ggtccggagg ctgctagaga caccctgtgt ccgggagact    1260
ttggcccggc acctctcccc acaagcccac catcgactgc acgctctgct tgccgcctgc    1320

tctacctcca tgctcatcct cttcaacctg gtgttcctcg ggggaattca agttgggaaa 1380
acctactgga acaggatctt cctgcaagca gccttcaga 1419

<210> 5
<211> 500
<212> PRT
<213> Drosophila

<400> 5

Met Ser Arg Leu Pro Asp Arg Ser Leu Leu Thr Arg Cys Glu Ile Phe
                5                   10                  15
Val Tyr Phe Gly Val Tyr Ile Ala Tyr Ile Val Val Gly Leu Tyr Lys
            20                  25                  30
Ile Tyr Gly Leu Arg Asp His Ile Val Lys Glu Ala Lys Phe Gln Phe
        35                  40                  45
Pro Glu Gly Trp Ser Leu Tyr Pro Phe Ser Gln Arg Arg Arg Asp Asp
    50                  55                  60
Ser Asn Asp Glu Leu Glu Asn Phe Gly Asp Phe Ile Val Ser Phe Trp
65                  70                  75                  80
Pro Phe Tyr Leu Leu His Val Ala Val Gln Gly Phe Ile Arg Trp Lys
                85                  90                  95
Arg Pro Arg Leu Gln Cys Leu Gly Phe Ile Gly Val Cys Ala Leu Ala
            100                 105                 110
Leu Ser Val Asn Leu Asp Trp Ser Ser Met Val Leu Leu Val Thr Leu
        115                 120                 125
Ile Ala Ser Tyr Tyr Ile Val Ser Leu Leu Ser Leu Lys Phe Leu Val
    130                 135                 140
Trp Leu Leu Ser Ala Gly Trp Ile Leu Cys Ile Asn Val Met Gln Lys
145                 150                 155                 160
Asn Val Trp Trp Thr Asp Arg Val Gly Tyr Thr Glu Tyr Val Leu Val
                165                 170                 175
Ile Val Thr Met Ser Trp Ser Val Leu Arg Gly Cys Ser Tyr Ser Leu
            180                 185                 190
Ser Lys Ile Gly Ala Lys Gln Glu Asp Leu Thr Arg Tyr Ser Leu Val
        195                 200                 205

Gln Tyr Leu Gly Tyr Ala Met Tyr Phe Pro Cys Leu Thr Tyr Gly Pro
    210                 215                 220
Ile Ile Ser Tyr Gln Arg Phe Ala Ala Arg Arg Glu Asp Glu Val Gln
225                 230                 235                 240
Asn Trp Leu Gly Phe Val Gly Gly Val Leu Arg Ser Ala Ile Trp Trp
                245                 250                 255
Leu Val Met Gln Cys Ala Leu His Tyr Phe Tyr Ile His Tyr Met Ser
            260                 265                 270
Arg Asp Val Arg Met Val Glu Met Met Asp Ser Val Phe Trp Gln His
        275                 280                 285
Ser Ala Gly Tyr Phe Met Gly Gln Phe Phe Phe Leu Tyr Tyr Val Val
    290                 295                 300
Thr Tyr Gly Leu Gly Ile Ala Phe Ala Val Gln Asp Gly Ile Pro Ala
305                 310                 315                 320
Pro Asn Arg Pro Arg Cys Ile Gly Arg Ile His Phe Tyr Ser Asp Met
                325                 330                 335
Trp Lys Tyr Phe Asp Glu Gly Leu Tyr Glu Phe Leu Phe Gln Asn Ile
            340                 345                 350
Tyr Ala Glu Leu Cys Gly Lys Arg Ser Ser Ala Ala Ala Lys Phe Gly
        355                 360                 365
Ala Thr Ala Leu Thr Phe Ala Phe Val Phe Val Trp His Gly Cys Tyr
    370                 375                 380
Thr Tyr Val Leu Ile Trp Ser Ile Leu Asn Phe Leu Cys Leu Ala Ala
385                 390                 395                 400
Glu Lys Val Phe Lys Thr Phe Thr Ala Met Pro Glu Tyr Gln Arg Trp
                405                 410                 415
Thr Gln Arg His Leu Gly Ala Val Gly Ala Gln Arg Leu Tyr Ala Met
            420                 425                 430
Leu Ala Thr Gln Leu Phe Ile Pro Ala Ala Phe Ser Asn Val Tyr Phe
        435                 440                 445
Ile Gly Gly Gln Glu Ile Gly Asp Phe Leu Met Arg Gly Ala Tyr Leu
    450                 455                 460
Ser Gly Val Gly Asn Tyr Val Ala Leu Cys Phe Cys Ser Tyr Cys Phe
465                 470                 475                 480
Phe Gln Cys Ser Glu Leu Leu Leu Thr Lys Ser Asp Gly Arg Ser Lys
                485                 490                 495

Thr Lys Thr Phe
            500

<210> 6
<211> 1500
<212> PRT
<213> Drosophila

<400> 6

atgtcaaggt taccagaccg atccctgctc acccgctgcg aaattttcgt ctacttcggg      60
gtctatatag cgtatatagt tgtgggcctg tacaagatct atgggctgcg ggatcacata     120
gtcaaggagg caaagttcca gtttccggag ggctggagtt tgtatccttt ttcccagcgg     180
cgtcgggatg acagcaacga tgagctggag aactttggag acttcatcgt cagcttttgg     240
cccttttact tgctgcacgt ggctgtccaa gtattcatac gctggaaacg tccccgcctt     300
cagtgtctgg gattcatcgg cgtctgtgcc ttggctctta gtgtaaacct ggactggtca     360
tcgatggtac tactagtcac attgatagcc agctactaca tcgtttccct gctctcacta     420
aaatttctcg tgtggctttt gtccgctggc tggatactgt gcatcaatgt gatgcaaaag     480
aatgtctggt ggacagatcg cgttggttac acggaatacg tcctggtgat tgtcacaatg     540
tcctggagtg tgcttcgcgg atgcagttat tccctgtcca agattggagc taagcaggag     600
gatcttacga gatacagcct cgtccagtat ctgggctacg ccatgtactt tccctgtctg     660
acctatgggc cgatcattag ctaccagagg ttcgcagccc ggagggagga tgaagtgcaa     720
aactggctgg gattcgtcgg aggagtcctg cgcagcgcca tatggtggtt ggtgatgcag     780
tgcgccctcc actatttcta tatacactac atgtcgcggg atgtgcgaat ggtggagatg     840
atggactcgg tgttttggca gcactccgcc ggctatttca tgggccagtt ctttttcctg     900
tactacgtgg tgacatacgg cttgggaata gcttttgctg tgcaggatgg catcccagcg     960
cccaatagac cgcgctgcat cggacgcatc cacttctact cggacatgtg gaagtacttc    1020
gatgagggtc tgtacgaatt ccttttccag aacatttacg cagagctgtg cggcaagcga    1080
tcatcggcag cggccaagtt cggtgccacc gcattgacct tcgcctttgt gttcgtctgg    1140
catggatgct acacctatgt gcttatctgg agcattctga acttcctctg cctggctgca    1200
gagaaggtgt ttaagacgtt taccgcgatg ccggagtacc agcggtggac acagcgtcat    1260
ctgggagccg tgggtgctca gcgcttgtac gcaatgctgg ccacccagct cttcattccg    1320
gccgccttct ccaatgtcta ctttatcggc ggacaggaga ttggagactt tctaatgcgt    1380
ggcgcctatc ttagtggagt gggcaactat gtggcactct gcttctgcag ctattgtttt    1440
tttcaatgct ctgagttgct cctgaccaag tcggatggtc gctccaagac caaacttttt    1500

<210> 7
<211> 28
<212> DNA
<213> Artificial Sequence

<220>
<223> Primer

<400> 7
ggggtaccat gctgctgctg gcgagatg                                          28

<210> 8
<211> 28
<212> DNA
<213> Artificial Sequence

<220>
<223> Primer

<400> 8
gctctagatc agctggactt gaccgcca                                          28

<210> 9
<211> 81
<212> DNA
<213> Artificial Sequence

<220>
<223> Primer

<400> 9
catgccatgg gcagcagcca tcatcatcat catcacagca gcggcgacga cgacgacaag   60
tgcggaccgg gcagggggtt c                                                 81

<210> 10
<211> 32

<212> DNA
<213> Artificial Sequence

<220>
<223> Primer

<400> 10
cgggatcctc agccgccgga tttggccgcc ac                                    32

<210> 11
<211> 28
<212> DNA
<213> Artificial Sequence

<220>
<223> Primer

<400> 11
ggggtaccat gccgccccga tgggaact                                         28

<210> 12
<211> 28
<212> DNA
<213> Artificial Sequence

<220>
<223> Primer

<400> 12
gctctagatt agtccgtggc gtaggtct                                         28

<210> 13
<211> 26
<212> DNA
<213> Artificial Sequence

<220>
<223> Primer

<400> 13
atgtcaaggt taccagaccg atccct                                          26

<210> 14
<211> 26
<212> DNA
<213> Artificial Sequence

<220>
<223> Primer

<400> 14
tcaaaaagtt ttggtcttgg agcgac                                          26

<210> 15
<211> 26
<212> DNA
<213> Artificial Sequence

<220>
<223> Primer

<400> 15
ggaattccac catgccgccc cgatgg                                          26

<210> 16
<211> 26
<212> DNA
<213> Artificial Sequence

<220>
<223> Primer

<400> 16
ggggtaccgt ccgtggcgta ggtctg                                          26

<210> 17
<211> 31
<212> DNA
<213> Artificial Sequence

<220>
<223> Primer

<400> 17
atttgcggcc gccaccatgt caaggttacc a                                    31

<210> 18
<211> 26
<212> DNA
<213> Artificial Sequence

<220>
<223> Primer

<400> 18
ggggtaccaa aagttttggt cttgga                                          26

<210> 19
<211> 21
<212> DNA
<213> Artificial Sequence

<220>
<223> DNA:RNA hybrid molecule

<400> 19
gggaagaucu ccagaaacut t                                               21

<210> 20
<211> 21
<212> DNA
<213> Artificial Sequence

<220>
<223> DNA:RNA hybrid molecule

<400> 20
ggaauuugag cuggagacut t                                                21

<210> 21
<211> 21
<212> DNA
<213> Artificial Sequence

<220>
<223> DNA:RNA hybrid molecule

<400> 21
uucuccgaac gugucacgut t                                                21

<210> 22
<211> 462
<212> PRT
<213> Homo sapience

<400> 22

<210> 23
<211> 1386
<212> DNA
<213> Homo sapience

<400> 23

atgctgctgc tggcgagatg tctgctgcta gtcctcgtct cctcgctgct ggtatgctcg     60
ggactggcgt gcggaccggg cagggggttc gggaagagga ggcaccccaa aaagctgacc    120
cctttagcct acaagcagtt tatccccaat gtggccgaga agaccctagg cgccagcgga    180
aggtatgaag ggaagatctc cagaaactcc gagcgattta aggaactcac ccccaattac    240
aaccccgaca tcatatttaa ggatgaagaa aacaccggag cggacaggct gatgactcag    300
aggtgtaagg acaagttgaa cgctttggcc atctcggtga tgaaccagtg gccaggagtg    360
aaactgcggg tgaccgaggg ctgggacgaa gatggccacc actcagagga gtctctgcac    420
tacgagggcc gcgcagtgga catcaccacg tctgaccgcg accgcagcaa gtacggcatg    480
ctggcccgcc tggcggtgga ggccggcttc gactgggtgt actacgagtc caaggcacat    540
atccactgct cggtgaaagc agagaactcg gtggcggcca aatcgggagg ctgcttcccg    600
ggctcggcca cggtgcacct ggagcagggc ggcaccaagc tggtgaagga cctgagcccc    660
ggggaccgcg tgctggcggc ggacgaccag ggccggctgc tctacagcga cttcctcact    720
ttcctggacc gcgacgacgg cgccaagaag gtcttctacg tgatcgagac gcgggagccg    780

cgcgagcgcc tgctgctcac cgccgcgcac ctgctctttg tggcgccgca caacgactcg 840
gccaccgggg agcccgaggc gtcctcgggc tcggggccgc cttccggggg cgcactgggg 900
cctcgggcgc tgttcgccag ccgcgtgcgc ccgggccagc gcgtgtacgt ggtggccgag 960
cgtgacgggg accgccggct cctgcccgcc gctgtgcaca gcgtgaccct aagcgaggag 1020
gccgcgggcg cctacgcgcc gctcacggcc cagggcacca ttctcatcaa ccgggtgctg 1080
gcctcgtgct acgcggtcat cgaggagcac agctgggcgc accgggcctt cgcgcccttc 1140
cgcctggcgc acgcgctcct ggctgcactg gcgcccgcgc gcacggaccg cggcggggac 1200
agcggcggcg gggaccgcgg gggcggcggc ggcagagtag ccctaaccgc tccaggtgct 1260
gccgacgctc cgggtgcggg ggccaccgcg ggcatccact ggtactcgca gctgctctac 1320
caaataggca cctggctcct ggacagcgag gccctgcacc cgctgggcat ggcggtcaag 1380
tccagc 1386